# EUROPEAN PATENT SPECIFICATION

(11) **EP 4 186 437 B1**
(45) Date of publication and mention of the grant of the patent: **22.05.2024**
(21) Application number: 22205743.2
(22) Date of filing: 07.11.2022
(51) Int. Cl.: A61B 8/08, G06T 7/00, A61B 8/00, G06T 7/12

(54) **ULTRASOUND IMAGE ANALYSIS APPARATUS, ULTRASOUND DIAGNOSTIC APPARATUS, AND CONTROL METHOD FOR ULTRASOUND IMAGE ANALYSIS APPARATUS**
ULTRASCHALLBILDANALYSEVORRICHTUNG, ULTRASCHALLDIAGNOSEVORRICHTUNG UND STEUERUNGSVERFAHREN FÜR ULTRASCHALLBILDANALYSEVORRICHTUNG
APPAREIL D'ANALYSE D'IMAGE ULTRASONORE, APPAREIL DE DIAGNOSTIC ULTRASONORE ET PROCÉDÉ DE COMMANDE POUR APPAREIL D'ANALYSE D'IMAGE ULTRASONORE

(30) Priority: 25.11.2021 JP 2021191286
(43) Date of publication of application: 31.05.2023
(73) Proprietor: FUJIFILM Corporation, Tokyo 106-8620 (JP)
(72) Inventor: KOSHINO, Riko, Tokyo, 106-8620 (JP)
(74) Representative: Hughes, Andrea Michelle

(56) References cited:
- US-A1- 2010 014 738
- US-A1- 2015 070 385
- US-A1- 2021 158 513
- RUEY-FENG CHANG ET AL: "Breast Density Analysis in 3-D Whole Breast Ultrasound Images", CONFERENCE PROCEEDINGS. ANNUAL INTERNATIONAL CONFERENCE OF THE IEEE ENGINEERING IN MEDICINE AND BIOLOGY SOCIETY (IEEE CAT. NO. 06CH37748); 30 AUG.-3 SEPT. 2006; NEW YORK, NY, USA, IEEE, PISCATAWAY, NJ, USA, 1 August 2006 (2006-08-01), pages 2795-2798, XP031171285, ISBN: 978-1-4244-0032-4

## Description

### BACKGROUND OF THE INVENTION

### 1. Field of the Invention

The present invention relates to an ultrasound image analysis apparatus that performs analysis on an ultrasound image obtained by imaging a breast of a subject.

In addition, the present invention also relates to an ultrasound diagnostic apparatus including the ultrasound image analysis apparatus and a control method for the ultrasound image analysis apparatus.

### 2. Description of the Related Art

In related art, an ultrasound diagnostic apparatus using ultrasound images has been put into practical use in a medical field. In general, an ultrasound diagnostic apparatus includes an ultrasound probe in which a transducer array is provided and an apparatus main body connected to the ultrasound probe. An ultrasound beam is transmitted from the ultrasound probe toward a subject, an ultrasound echo from the subject is received by the ultrasound probe, and a reception signal is electrically processed. Thereby, an ultrasound image is generated.

A composition of a fat tissue and a mammary gland tissue in a breast varies from person to person, while an anatomical structure of a breast is common. In the mammary gland tissue, a main mammary duct branches into extralobular mammary ducts, which are connected to numerous lobules. A stroma is present around the lobules, and the mammary gland tissue includes the stroma. It is known that there are two types of stromata around lobules: a peripheral stroma and an edematous stroma. The peripheral stroma is present along a structure from the lobule to the mammary duct, and includes many collagen fibers. On the other hand, the edematous stroma fills a space between the peripheral stromata. In the edematous stroma, substrates are rich, and collagen fibers, fat, and the like coexist. The edematous stroma includes less collagen fibers as compared with the peripheral stroma.

In recent years, a concept of risk management for individual patients has spread. On the other hand, it is known that a ratio of a mammary gland region in a breast, particularly, a high density of mammary glands, is a cancer risk factor. The ratio of the mammary gland region in a breast can be measured by using a mammography apparatus.

In addition, in Su Hyun Lee et al. "Glandular Tissue Component and Breast Cancer Risk in Mammographically Dense Breasts at Screening Breast US", Radiology, Volume 301, October 1, 2021, it is reported that a cancer is likely to occur in a case where a ratio of a glandular tissue component (GTC) region including mammary ducts, lobules, and peripheral stromata in the mammary gland region is high even though the mammary gland region is almost the same. That is, in addition to the ratio of the mammary gland region in the breast, a ratio of the GTC region in the mammary gland region can be a risk factor. This implies a higher risk in patients with less advanced lobular retraction.

However, in the mammography apparatus, the peripheral stroma and the edematous stroma cannot be distinguished, and the entire mammary gland tissue is observed as whitish. As a result, a ratio of the GTC region in the mammary gland region cannot be measured.

JP2020-18694A discloses an ultrasound diagnostic apparatus that extracts a mammary gland region by detecting a boundary in a depth direction of an ultrasound image and detects a lesion portion existing in the mammary gland region. US2010/014738 discloses an ultrasound diagnostic apparatus that extracts a mammary gland region, determines the total volume of glandular tissue and calculates the absolute glandular tissue amount of the breast from the glandular volume.

### SUMMARY OF THE INVENTION

However, the ultrasound diagnostic apparatus disclosed in JP2020-18694A aims to detect a lesion portion in the mammary gland region, and is not interested in dividing the mammary gland region into finer tissues. As a result, there is a problem that a cancer risk of the mammary gland region cannot be estimated.

The present invention has been made to solve such problems in the related art, and an object of the present invention is to provide an ultrasound image analysis apparatus capable of estimating a cancer risk of a mammary gland region based on an ultrasound image.

Another object of the present invention is to provide an ultrasound diagnostic apparatus including such an ultrasound image analysis apparatus and a control method for an ultrasound image analysis apparatus.

In order to achieve the above objects, according to an aspect of the present invention, there is provided an ultrasound image analysis apparatus including: a mammary gland region detection unit that detects a mammary gland region from an ultrasound image obtained by imaging a breast of a subject; a glandular tissue component region extraction unit that extracts a glandular tissue component region including mammary ducts, lobules, and peripheral stromata in the mammary gland region detected by the mammary gland region detection unit; and a glandular tissue component region ratio calculation unit that calculates a ratio of the glandular tissue component region to the mammary gland region.

The mammary gland region detection unit may detect the mammary gland region by image recognition of the ultrasound image. In this case, the ultrasound image analysis apparatus may further include a breast region detection unit that detects a breast region located between a skin and a pectoralis major muscle from the ultrasound image. The mammary gland region detection unit may recognize a front boundary line and a rear boundary line in the breast region detected by the breast region detection unit, and may detect a region between the front boundary line and the rear boundary line, as the mammary gland region.

Alternatively, the mammary gland region detection unit may detect the mammary gland region from the ultrasound image by using deep learning.

The glandular tissue component region extraction unit may extract the glandular tissue component region by binarizing the mammary gland region of the ultrasound image by using a brightness threshold value.

The glandular tissue component region extraction unit may extract the glandular tissue component region from the mammary gland region of the ultrasound image by using deep learning.

The glandular tissue component region ratio calculation unit may calculate the ratio of the glandular tissue component region to the mammary gland region based on the number of occupied pixels of the mammary gland region and the number of occupied pixels of the glandular tissue component region in the ultrasound image.

The mammary gland region detection unit may detect the mammary gland region from each of a plurality of the ultrasound images obtained by imaging the breast of the subject at a plurality of predetermined locations of the breast of the subject.

The glandular tissue component region extraction unit may extract the glandular tissue component region from each of the mammary gland regions of the plurality of ultrasound images.

The glandular tissue component region ratio calculation unit may calculate the ratio of the glandular tissue component region to the mammary gland region in each of the plurality of ultrasound images.

The glandular tissue component region ratio calculation unit may calculate an average value of ratios of the glandular tissue component regions to the mammary gland regions in the plurality of ultrasound images.

The ultrasound image may be a three-dimensional ultrasound image, and the glandular tissue component region ratio calculation unit may calculate the ratio of the glandular tissue component region to the mammary gland region based on a volume of the mammary gland region detected by the mammary gland region detection unit and a volume of the glandular tissue component region extracted by the glandular tissue component region extraction unit.

According to another aspect of the present invention, there is provided an ultrasound diagnostic apparatus including: a monitor that displays an ultrasound image obtained by imaging a breast of a subject; and the ultrasound image analysis apparatus, in which the mammary gland region detection unit detects the mammary gland region from the ultrasound image, and the glandular tissue component region ratio calculation unit displays the calculated ratio of the glandular tissue component region to the mammary gland region on the monitor.

Further, the ultrasound diagnostic apparatus may further include a mammary gland region ratio calculation unit that calculates a ratio of the mammary gland region to the breast region and displays the calculated ratio on the monitor. In this case, the glandular tissue component region extraction unit may extract the glandular tissue component region in the mammary gland region only in a case where the ratio of the mammary gland region calculated by the mammary gland region ratio calculation unit is higher than a set value.

Preferably, the glandular tissue component region extraction unit extracts the glandular tissue component region by binarizing the mammary gland region of the ultrasound image by using a brightness threshold value, and displays a binarized image obtained by binarizing the mammary gland region on the monitor.

The brightness threshold value may be a constant value.

The glandular tissue component region extraction unit may detect an edge of the glandular tissue component region in the ultrasound image, and may automatically set the brightness threshold value based on a change in brightness value at the detected edge.

The ultrasound diagnostic apparatus may further include: a histogram creation unit that creates a histogram of brightness of the mammary gland region in the ultrasound image and displays the created histogram on the monitor. The brightness threshold value may be set by a user based on the histogram, the binarized image, and the ultrasound image which are displayed on the monitor.

The ultrasound diagnostic apparatus may further include: a breast schematic diagram generation unit that generates a schematic diagram of the breast on which a plurality of predetermined locations are plotted and displays the schematic diagram on the monitor.

The ultrasound diagnostic apparatus may further include: an imaging guide unit that guides a user to perform imaging of the ultrasound images at the plurality of predetermined locations.

Preferably, the glandular tissue component region ratio calculation unit stores the calculated ratio of the glandular tissue component region in a tag associated with the ultrasound image.

The glandular tissue component region ratio calculation unit may display, on the monitor, a past ratio of the glandular tissue component region that is calculated based on the past ultrasound image of the subject, together with a latest ratio of the glandular tissue component region that is calculated based on the latest ultrasound image of the subject.

Preferably, the ultrasound diagnostic apparatus further includes: an ultrasound probe; and an image generation unit that generates the ultrasound image obtained by imaging the breast of the subject by transmitting and receiving an ultrasound beam to and from the subject using the ultrasound probe.

According to still another aspect of the present invention, there is provided a control method for an ultrasound image analysis apparatus, the method including: detecting a mammary gland region from an ultrasound image obtained by imaging a breast of a subject; extracting a glandular tissue component region including mammary ducts, lobules, and peripheral stromata in the mammary gland region; and calculating a ratio of the glandular tissue component region to the mammary gland region.

According to the present invention, it is possible to estimate a cancer risk of a mammary gland region by detecting, via a mammary gland region detection unit, a mammary gland region from an ultrasound image obtained by imaging a breast of a subject, extracting, via a glandular tissue component region extraction unit, a glandular tissue component region including mammary ducts, lobules, and peripheral stromata in the mammary gland region, and calculating, via a glandular tissue component region ratio calculation unit, a ratio of the glandular tissue component region to the mammary gland region.

### BRIEF DESCRIPTION OF THE DRAWINGS

Fig. 1 is a block diagram illustrating a configuration of an ultrasound diagnostic apparatus according to an embodiment 1 of the present invention.
Fig. 2 is a block diagram illustrating an internal configuration of a transmission/reception circuit according to the embodiment 1.
Fig. 3 is a block diagram illustrating an internal configuration of an image generation unit according to the embodiment 1.
Fig. 4 is a diagram illustrating an ultrasound image obtained by imaging a mammary gland region of a subject.
Fig. 5 is a diagram illustrating an ultrasound image including a mammary gland region in which a GTC region is imaged.
Fig. 6 is a diagram illustrating a binarized image obtained by binarizing the mammary gland region by using a brightness threshold value.
Fig. 7 is a flowchart illustrating an operation according to the embodiment 1.
Fig. 8 is a block diagram illustrating a configuration of an ultrasound diagnostic apparatus according to an embodiment 2.
Fig. 9 is a flowchart illustrating an operation according to the embodiment 2.
Fig. 10 is a block diagram illustrating a configuration of an ultrasound diagnostic apparatus according to an embodiment 3.
Fig. 11 is a flowchart illustrating an operation according to the embodiment 3.
Fig. 12 is a block diagram illustrating a configuration of an ultrasound diagnostic apparatus according to an embodiment 4.
Fig. 13 is a diagram illustrating a configuration of a breast schematic diagram generated in the embodiment 4.
Fig. 14 is a diagram illustrating a breast schematic diagram with a probe mark.
Fig. 15 is a flowchart illustrating an operation according to the embodiment 4.
Fig. 16 is a block diagram illustrating a configuration of an ultrasound diagnostic apparatus according to an embodiment 5.
Fig. 17 is diagram illustrating a breast schematic diagram and an imaging guide displayed on a monitor in the embodiment 5.
Fig. 18 is diagram illustrating another breast schematic diagram and an imaging guide displayed on a monitor in the embodiment 5.
Fig. 19 is a flowchart illustrating an operation according to the embodiment 5.

### DESCRIPTION OF THE PREFERRED EMBODIMENTS

Hereinafter, embodiments of the present invention will be described with reference to the accompanying drawings.

A description of components to be described below is based on a representative embodiment of the present invention. On the other hand, the present invention is not limited to such an embodiment.

Note that, in this specification, a numerical range represented by using "to" means a range including numerical values described before and after "to", both ends inclusive, as a lower limit value and an upper limit value.

In this specification, it is assumed that terms "identical" and "same" include an error margin which is generally allowed in the technical field.

### Embodiment 1

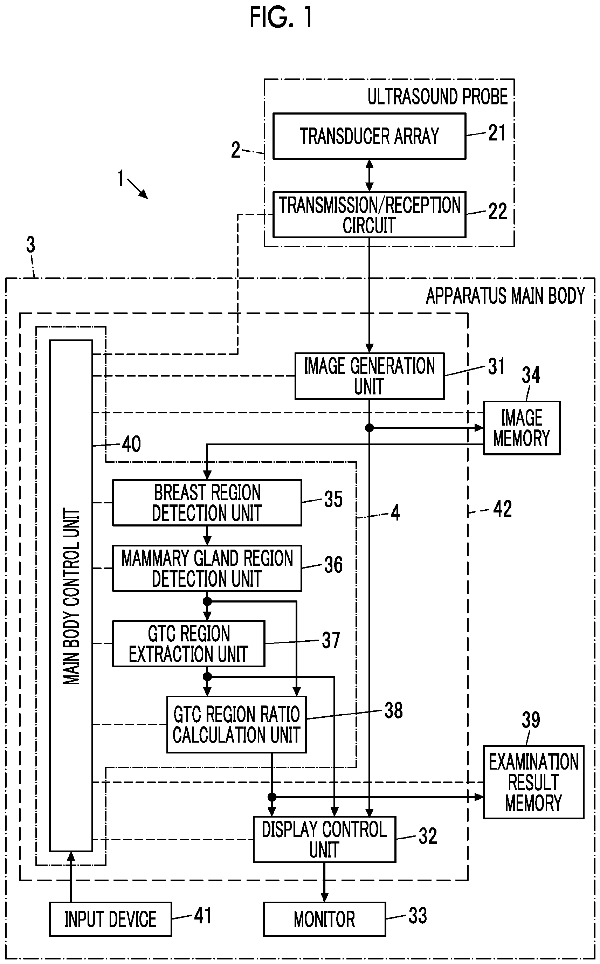

Fig. 1 illustrates a configuration of an ultrasound diagnostic apparatus 1 according to an embodiment 1 of the present invention. The ultrasound diagnostic apparatus 1 includes an ultrasound probe 2 and an apparatus main body 3. The ultrasound probe 2 and the apparatus main body 3 are wired-connected to each other via a cable (not illustrated).

The ultrasound probe 2 includes a transducer array 21 and a transmission/reception circuit 22 connected to the transducer array 21.

The apparatus main body 3 includes an image generation unit 31 connected to the transmission/reception circuit 22 of the ultrasound probe 2. A display control unit 32 and a monitor 33 are sequentially connected to the image generation unit 31, and an image memory 34 is connected to the image generation unit 31. In addition, a breast region detection unit 35, a mammary gland region detection unit 36, a glandular tissue component (GTC) region extraction unit 37, and a GTC region ratio calculation unit 38 are sequentially connected to the image memory 34, and the GTC region extraction unit 37 and the GTC region ratio calculation unit 38 are connected to the display control unit 32. In addition, the mammary gland region detection unit 36 is connected to the GTC region extraction unit 37.

Further, an examination result memory 39 is connected to the GTC region ratio calculation unit 38.

A main body control unit 40 is connected to the image generation unit 31, the display control unit 32, the image memory 34, the breast region detection unit 35, the mammary gland region detection unit 36, the GTC region extraction unit 37, the GTC region ratio calculation unit 38, and the examination result memory 39. An input device 41 is connected to the main body control unit 40. In addition, the transmission/reception circuit 22 of the ultrasound probe 2 is connected to the main body control unit 40.

A processor 42 is configured by the image generation unit 31, the display control unit 32, the breast region detection unit 35, the mammary gland region detection unit 36, the GTC region extraction unit 37, the GTC region ratio calculation unit 38, and the main body control unit 40.

Further, an ultrasound image analysis apparatus 4 is configured by the breast region detection unit 35, the mammary gland region detection unit 36, the GTC region extraction unit 37, the GTC region ratio calculation unit 38, and the main body control unit 40 in the processor 42.

The transducer array 21 of the ultrasound probe 2 includes a plurality of ultrasound transducers which are one-dimensionally or two-dimensionally arranged. Each of these transducers transmits an ultrasound wave according to a drive signal supplied from the transmission/reception circuit 22, receives a reflected wave from a subject, and outputs an analog reception signal. Each transducer is configured by, for example, forming electrodes on both ends of a piezoelectric body such as a piezoelectric ceramic represented by lead zirconate titanate (PZT), a polymeric piezoelectric element represented by poly vinylidene di fluoride (PVDF), or a piezoelectric single crystal represented by a lead magnesium niobate-lead titanate solid solution (PMN-PT).

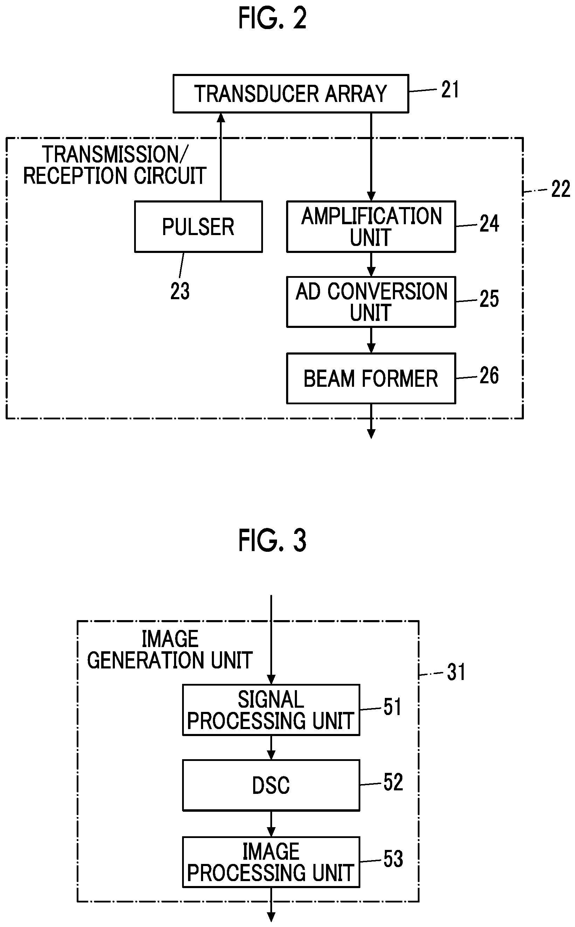

The transmission/reception circuit 22 transmits an ultrasound wave from the transducer array 21 and generates a sound ray signal based on the reception signal acquired by the transducer array 21 under a control of the main body control unit 40. As illustrated in Fig. 2, the transmission/reception circuit 22 includes a pulser 23 connected to the transducer array 21, an amplification unit 24 sequentially connected in series to the transducer array 21, an analog digital (AD) conversion unit 25, and a beam former 26.

The pulser 23 includes, for example, a plurality of pulse generators, adjusts a delay amount of each drive signal based on a transmission delay pattern which is selected according to a control signal from the main body control unit 40 such that ultrasound waves to be transmitted from the plurality of transducers of the transducer array 21 form ultrasound beams, and supplies each drive signal with the adjusted delay amount to the plurality of transducers. In this way, in a case where a voltage having a pulse shape or a continuous wave shape is applied to the electrodes of the transducers of the transducer array 21, the piezoelectric body expands and contracts. Thereby, ultrasound waves having a pulse shape or a continuous wave shape are generated from each transducer, and thus an ultrasound beam is formed from a composite wave of these ultrasound waves.

The transmitted ultrasound beam is reflected by an object such as a portion of a subject, and an ultrasound echo propagates toward the transducer array 21 of the ultrasound probe 2. The ultrasound echo which propagates toward the transducer array 21 in this way is received by each transducer included in the transducer array 21. At this time, in a case where the propagating ultrasound echo is received, each transducer included in the transducer array 21 expands and contracts. Thereby, a reception signal as an electrical signal is generated, and these reception signals are output to the amplification unit 24.

The amplification unit 24 amplifies the signal which is input from each transducer included in the transducer array 21, and transmits the amplified signal to the AD conversion unit 25. The AD conversion unit 25 converts the signal transmitted from the amplification unit 24 into pieces of digital reception data, and transmits the pieces of reception data to the beam former 26. The beam former 26 performs so-called reception focus processing by applying and adding a delay to each of the pieces of reception data which is converted by the AD conversion unit 25 according to a sound velocity or a sound velocity distribution which is set based on a reception delay pattern selected according to a control signal from the main body control unit 40. By this reception focus processing, a sound ray signal obtained by performing phasing addition on each of the pieces of reception data which is converted by the AD conversion unit 25 and narrowing down a focus of the ultrasound echo is acquired.

As illustrated in Fig. 3, the image generation unit 31 of the apparatus main body 3 has a configuration in which a signal processing unit 51, a digital scan converter (DSC) 52, and an image processing unit 53 are connected in series.

The signal processing unit 51 performs, on the sound ray signal transmitted from the transmission/reception circuit 22 of the ultrasound probe 2, correction of attenuation due to a distance according to a depth of a reflection position of the ultrasound wave and then performs envelope detection processing. Thereby, an ultrasound image signal (B-mode image signal), which is tomographic image information related to tissues in the subject, is generated.

The DSC 52 converts (raster-converts) the ultrasound image signal generated by the signal processing unit 51 into an image signal conforming to a normal television signal scanning method.

The image processing unit 53 performs various required image processing such as gradation processing on the ultrasound image signal which is input from the DSC 52, and then outputs a signal representing the ultrasound image to the display control unit 32 and the image memory 34. The signal representing the ultrasound image generated by the image generation unit 31 in this way is simply referred to as an ultrasound image.

Under a control of the main body control unit 40, the display control unit 32 performs predetermined processing on the ultrasound image transmitted from the image generation unit 31, and displays the ultrasound image on the monitor 33.

The monitor 33 displays the ultrasound image under a control of the display control unit 32, and includes a display device such as a liquid crystal display (LCD) or an organic electroluminescence (EL) display.

The image memory 34 is a memory that stores the ultrasound image generated by the image generation unit 31 under a control of the main body control unit 40. For example, the image memory 34 can store a plurality of frames of ultrasound images generated by the image generation unit 31 in correspondence with diagnosis on a mammary gland region of a breast of a subject.

As the image memory 34, a flash memory, a hard disc drive (HDD), a solid state drive (SSD), a flexible disc (FD), a magneto-optical (MO) disc, a magnetic tape (MT), a random access memory (RAM), a compact disc (CD), a digital versatile disc (DVD), a secure digital (SD) card, and a recording medium such as a Universal Serial Bus (USB) memory can be used.

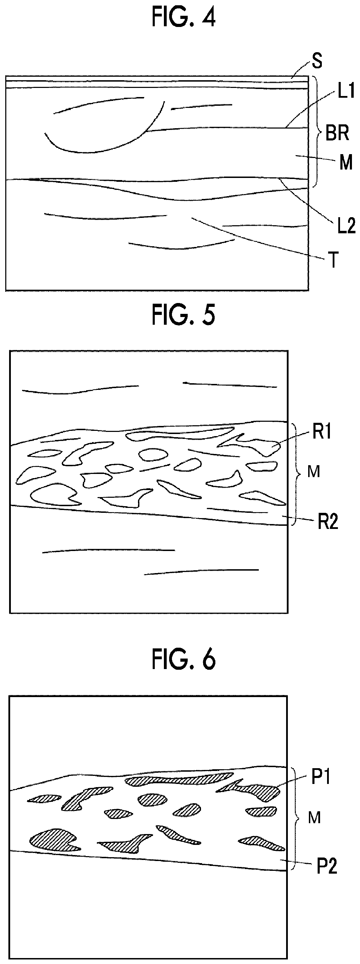

The breast region detection unit 35 detects a breast region of the subject from the ultrasound image generated by the image generation unit 31. Fig. 4 illustrates an example of an ultrasound image obtained by imaging a breast of a subject. The ultrasound image is a tomographic image obtained by bringing a tip of the ultrasound probe 2 into contact with the breast of the subject and performing imaging. A skin S of the subject appears in an upper end of the ultrasound image representing the shallowest portion, and a pectoralis major muscle T appears in a lower portion of the ultrasound image representing a deeper portion. The breast region detection unit 35 can recognize the skin S and the pectoralis major muscle T from the ultrasound image, and detect a deep region between the skin S and the pectoralis major muscle T, as a breast region BR.

The mammary gland region detection unit 36 detects a mammary gland region of the subject from the ultrasound image generated by the image generation unit 31. As illustrated in Fig. 4, the mammary gland region detection unit 36 can recognize a front boundary line L1 located on a shallower side and a rear boundary line L2 located on a deeper side in the breast region BR detected by the breast region detection unit 35, and detect a deep region between the front boundary line L1 and the rear boundary line L2, as a mammary gland region M.

In order to detect the breast region BR and the mammary gland region M described above, image recognition can be performed using at least one of template matching, an image analysis technique using feature amounts such as adaptive boosting (Adaboost), support vector machine (SVM), or scale-invariant feature transform (SIFT), or a determination model learned by using a machine learning technique such as deep learning.

Note that the determination model is a learned model obtained by learning the breast region BR and the mammary gland region M (segmentation) of the breast region BR in a learning ultrasound image obtained by imaging the breast.

The GTC region extraction unit 37 extracts a GTC region from the mammary gland region M of the subject that is detected by the mammary gland region detection unit 36. The GTC region includes mammary ducts, lobules, and peripheral stromata in the mammary gland region M, and an edematous stroma fills a space between the peripheral stromata. In the edematous stroma, substrates are rich and adipocytes coexist. For this reason, in a case where the mammary gland region M is observed in an ultrasound image, the edematous stroma has a high echo level and appears with high brightness. On the other hand, the mammary ducts, the lobules, and the peripheral stromata that are included in the GTC region have relatively low echo levels, and have brightness lower than the brightness of the edematous stroma.

For this reason, the GTC region extraction unit 37 binarizes the mammary gland region M of the ultrasound image by using, for example, a brightness threshold value Th. Thus, the GTC region and the edematous stroma in the mammary gland region M are distinguished from each other. Thereby, the GTC region can be extracted.

For example, as in the ultrasound image illustrated in Fig. 5, in a case where the GTC region R1 and the edematous region R2 filled with the edematous stroma coexist in the mammary gland region M, by binarizing the mammary gland region M by using an appropriate brightness threshold value Th, a binarized image as illustrated in Fig. 6 is obtained.

In the binarized image of Fig. 6, pixels having brightness values lower than the brightness threshold value Th are represented in black (a shaded regions in Fig. 6), and the pixels form a black portion P1. In addition, pixels having brightness values equal to or higher than the brightness threshold value Th are represented in white, and the pixels form a white portion P2. The black portion P1 corresponds to the GTC region R1, and the white portion P2 corresponds to the edematous region R2. That is, by binarizing the mammary gland region M, the GTC region R1 can be extracted as the black portion P1.

The binarized image created by the GTC region extraction unit 37 is displayed on the monitor 33 via the display control unit 32.

Note that a predetermined constant value can be used as the brightness threshold value Th.

In addition, the GTC region extraction unit 37 may perform edge detection on the GTC region R1 of the ultrasound image by image analysis, and automatically calculate a brightness threshold value Th based on a change in brightness values of a detected edge portion, that is, a change in brightness values of a plurality of pixels from the inside to the outside of the GTC region R1. In this way, it is possible to automatically set the brightness threshold value Th suitable for the ultrasound image as an image analysis target, and to acquire a binarized image suitable for the ultrasound image.

Further, the GTC region extraction unit 37 can also extract the GTC region R1 by using a determination model which is learned using a machine learning technique such as deep learning. In this case, a learned model obtained by learning the GTC region R1 (segmentation) of the mammary gland region M in the learning ultrasound image obtained by imaging the breast is used as the determination model.

The GTC region ratio calculation unit 38 calculates a ratio of the GTC region R1 to the mammary gland region M, and displays the calculated ratio on the monitor 33. Specifically, the GTC region ratio calculation unit 38 receives the mammary gland region M detected by the mammary gland region detection unit 36 and the GTC region R1 extracted by the GTC region extraction unit 37, and calculates a GTC region ratio of the GTC region R1 to the mammary gland region M.

In addition, the GTC region ratio calculation unit 38 stores the calculated GTC region ratio in the examination result memory 39, as an examination result.

The GTC region ratio cannot be measured by a mammography apparatus. On the other hand, in the ultrasound diagnostic apparatus according to the embodiment 1, the GTC region ratio can be calculated based on, for example, the number of occupied pixels of the mammary gland region M and the number of occupied pixels of the GTC region R1 in the ultrasound image. Specifically, the GTC region ratio is represented by a ratio of a sum of the number of occupied pixels of all the GTC regions R1 existing in the mammary gland region M to the number of occupied pixels of the entire mammary gland region M.

The GTC region ratio calculation unit 38 may display the calculated GTC region ratio on the monitor 33 by using a numerical value, or may display the calculated GTC region ratio on the monitor 33 by using a pie graph, a bar graph, or the like.

The examination result memory 39 is a memory that stores, as an examination result, the GTC region ratio which is calculated by the GTC region ratio calculation unit 38 under a control of the main body control unit 40.

Similar to the image memory 34, as the examination result memory 39, a recording medium such as a flash memory, an HDD, an SSD, an FD, an MO disc, an MT, a RAM, a CD, a DVD, an SD card, a USB memory, or the like can be used.

Note that the examination result memory 39 may be either a memory integrated with the image memory 34 or a memory separated from the image memory 34.

In addition, the ultrasound image generated by the image generation unit 31 is represented, for example, as image data with a so-called digital imaging and communications in medicine (DICOM) format in which patient identification information is added. The ultrasound image includes a tag for storing accessory information, and the GTC region ratio which is calculated by the GTC region ratio calculation unit 38 can be stored with a tag associated with the ultrasound image.

The main body control unit 40 controls each unit of the apparatus main body 3 and the transmission/reception circuit 22 of the ultrasound probe 2 based on a control program or the like which is stored in advance.

In addition, a main-body-side storage unit (not illustrated) is connected to the main body control unit 40. The main-body-side storage unit stores a control program or the like. In addition, as the main-body-side storage unit, for example, a flash memory, a RAM, an SD card, an SSD, or the like can be used.

The input device 41 is a device that allows a user to perform an input operation, and includes, for example, devices such as a keyboard, a mouse, a trackball, a touch pad, and a touch sensor overlapped and provided on the monitor 33.

Note that the processor 42 including the image generation unit 31, the display control unit 32, the breast region detection unit 35, the mammary gland region detection unit 36, the GTC region extraction unit 37, the GTC region ratio calculation unit 38, and the main body control unit 40 is configured with a central processing unit (CPU) and a control program for causing the CPU to perform various processing. The processor 42 may be configured by using a field programmable gate array (FPGA), a digital signal processor (DSP), an application specific integrated circuit (ASIC), a graphics processing unit (GPU), or other integrated circuits (IC), or may be configured by using a combination thereof.

In addition, the image generation unit 31, the display control unit 32, the breast region detection unit 35, the mammary gland region detection unit 36, the GTC region extraction unit 37, the GTC region ratio calculation unit 38, and the main body control unit 40 of the processor 42 can be partially or entirely integrated into one CPU or the like.

The ultrasound image analysis apparatus 4 that includes the breast region detection unit 35, the mammary gland region detection unit 36, the GTC region extraction unit 37, the GTC region ratio calculation unit 38, and the main body control unit 40 of the processor 42 may be configured with an FPGA, a DSP, an ASIC, a GPU, or other ICs independently from the image generation unit 31 and the display control unit 32.

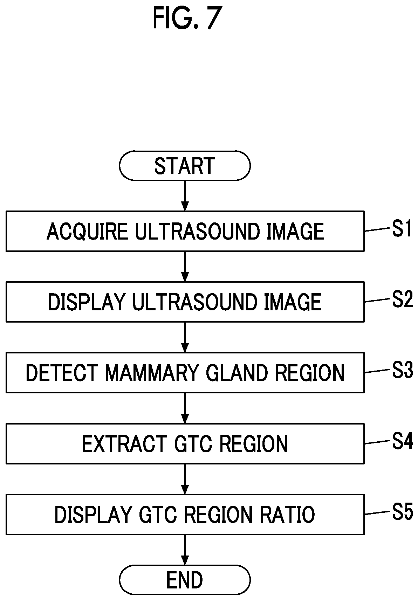

Next, an operation of the ultrasound diagnostic apparatus 1 according to the embodiment 1 will be described with reference to a flowchart illustrated in Fig. 7.

First, in step S1, a breast of a subject is imaged by using the ultrasound probe 2, and thus an ultrasound image is acquired. At this time, under a control of the main body control unit 40, transmission and reception of ultrasound waves from the plurality of transducers of the transducer array 21 are started according to a drive signal from the pulser 23 of the transmission/reception circuit 22 of the ultrasound probe 2. The ultrasound echo from the inside of the breast of the subject is received by the plurality of transducers of the transducer array 21. The reception signal which is an analog signal is output to the amplification unit 24, and is amplified by the amplification unit 24. The amplified reception signal is AD-converted by the AD conversion unit 25, and thus the reception data is acquired.

The reception focus processing is performed on the reception data by the beam former 26, and the sound ray signal generated by the reception focusing processing is transmitted to the image generation unit 31 of the apparatus main body 3. An ultrasound image representing tomographic image information of the breast of the subject is generated by the image generation unit 31. At this time, attenuation correction according to a depth of a reflection position of the ultrasound wave and envelope detection processing are performed on the sound ray signal by the signal processing unit 51 of the image generation unit 31. The sound ray signal is converted into an image signal conforming to a scanning method of a normal television signal by the DSC 52, and various required image processing such as gradation processing is performed on the image signal by the image processing unit 53.

Subsequently, in step S2, the ultrasound image generated by the image generation unit 31 is displayed on the monitor 33 via the display control unit 32, and is stored in the image memory 34.

Note that, in a case where an ultrasound image is acquired, under a control of the main body control unit 40, a transmission intensity of the ultrasound wave and a depth range of the ultrasound image displayed on the monitor 33 are adjusted such that the entire breast of the subject, that is, for example, the deep portion between the skin S and the pectoralis major muscle T of the subject illustrated in Fig. 4, is fitted into the screen.

In a case where the ultrasound image is stored in the image memory 34 in this way, in step S3, the ultrasound image is input to the ultrasound image analysis apparatus 4, and a breast region BR of the subject is detected from the ultrasound image by the breast region detection unit 35. For example, as illustrated in Fig. 4, the deep region between the skin S of the subject appearing in the shallowest portion of the ultrasound image and the pectoralis major muscle T appearing in the deeper portion is detected as the breast region BR.

Further, the ultrasound image is input to the mammary gland region detection unit 36 via the breast region detection unit 35. The mammary gland region detection unit 36 recognizes the front boundary line L1 and the rear boundary line L2 in the breast region BR detected by the breast region detection unit 35, and detects, as the mammary gland region M, the deep region between the front boundary line L1 and the rear boundary line L2.

Next, in step S4, the GTC region R1 is extracted from the mammary gland region M by the GTC region extraction unit 37 of the ultrasound image analysis apparatus 4, the mammary gland region M being detected by the mammary gland region detection unit 36. At this time, the GTC region extraction unit 37 binarizes the mammary gland region M of the ultrasound image illustrated in Fig. 5 by using, for example, a brightness threshold value Th as a predetermined constant value, and thus a binarized image as illustrated in Fig. 6 is acquired. In the binarized image of Fig. 6, the black portion P1 corresponds to the extracted GTC region R1, and the white portion P2 corresponds to the edematous region R2 filled with the edematous stroma.

The binarized image created by the GTC region extraction unit 37 is displayed on the monitor 33 via the display control unit 32.

Subsequently, in step S5, the GTC region ratio of the GTC region R1 to the mammary gland region M is calculated by the GTC region ratio calculation unit 38 of the ultrasound image analysis apparatus 4, and the GTC region ratio is displayed on the monitor 33 via the display control unit 32. At this time, the GTC region ratio calculation unit 38 can calculate the GTC region ratio based on a ratio of the number of occupied pixels of all the GTC regions R1 existing in the mammary gland region M to the number of occupied pixels of the entire mammary gland region M in the ultrasound image.

The calculated GTC region ratio is displayed on the monitor 33, as a numerical value, a pie graph, a bar graph, or the like, and is also stored in the examination result memory 39, as an examination result. Further, the calculated GTC region ratio can be stored in a tag with a DICOM format by being associated with the ultrasound image, or can be directly transmitted from the ultrasound diagnostic apparatus 1 to an external diagnostic report system or the like via a network.

In this way, the GTC region ratio that cannot be measured by the mammography apparatus is displayed on the monitor 33.

By checking the GTC region ratio displayed on the monitor 33, it is possible to estimate a cancer risk of the mammary gland region of the subject.

In addition, by utilizing a so-called picture archiving and communication system (PACS, a medical image management system) or the like, the DICOM in past examinations of the same subject may be acquired, and the past GTC region ratio stored in the DICOM may be obtained. In this case, the GTC region ratio calculation unit 38 can display the past GTC region ratio on the monitor 33 together with the latest GTC region ratio which is calculated based on the latest ultrasound image of the subject.

In a case where the GTC region ratio is not calculated in the past examination, based on the past ultrasound image acquired in the past examination, the past GTC region ratio may be calculated by the breast region detection unit 35, the mammary gland region detection unit 36, the GTC region extraction unit 37, and the GTC region ratio calculation unit 38, and the calculated past GTC region ratio may be displayed on the monitor 33 together with the latest GTC region ratio.

In the embodiment 1 described above, the ultrasound image generated by the image generation unit 31 is a two-dimensional ultrasound image. On the other hand, the image generation unit 31 can also generate a three-dimensional ultrasound image of the breast of the subject. After acquiring a plurality of two-dimensional ultrasound images on a plurality of different tomographic planes by performing planar scanning on the subject by using the ultrasound probe 2, a three-dimensional ultrasound image may be generated based on the plurality of two-dimensional ultrasound images. Alternatively, by using a three-dimensional probe instead of the ultrasound probe 2, a three-dimensional ultrasound image may be generated in a state where the three-dimensional probe is stationary.

The mammary gland region M is detected from the three-dimensional ultrasound image by the mammary gland region detection unit 36, and the GTC region R1 is extracted from the mammary gland region M of the three-dimensional ultrasound image by the GTC region extraction unit 37. The GTC region ratio calculation unit 38 can calculate a GTC region ratio of the GTC region R1 to the mammary gland region M based on a volume of the mammary gland region M detected by the mammary gland region detection unit 36 and a volume of the GTC region R1 extracted by the GTC region extraction unit 37. The calculated GTC region ratio is displayed on the monitor 33, and is stored in the examination result memory 39.

By calculating the GTC region ratio based on the three-dimensional ultrasound image in this way, it is possible to improve accuracy of estimation of a cancer risk of the mammary gland region M of the subject, and perform diagnosis with higher reliability.

In the embodiment 1 described above, as an example of the ultrasound diagnostic apparatus 1, the configuration including the ultrasound probe 2 and the apparatus main body 3 has been described. On the other hand, the example of the ultrasound diagnostic apparatus including the ultrasound image analysis apparatus 4 is not limited thereto. The ultrasound image analysis apparatus 4 can also be applied to an automatic breast ultrasound apparatus including a transducer assembly and a main body that automatically move while the breast is fixed, such as US2015/0094587A.

Further, in the embodiment 1 described above, the ultrasound image analysis apparatus 4 is provided inside the ultrasound diagnostic apparatus 1. On the other hand, the present invention is not limited thereto. The ultrasound image analysis apparatus 4 can be used separately from the ultrasound diagnostic apparatus 1. For example, the ultrasound image analysis apparatus 4 may be provided inside a server or the like (not illustrated) connected to a general-purpose ultrasound imaging apparatus via a network, an ultrasound image obtained by imaging the breast of the subject may be transmitted to the server by the general-purpose ultrasound imaging apparatus, and the GTC region ratio may be calculated by the ultrasound image analysis apparatus 4. Further, for example, as described in JP2008-161283A, JP2019-69319A, and the like, the ultrasound image analysis apparatus 4 may be provided in a mammography apparatus having an ultrasound measurement function, and the GTC region ratio can be calculated inside the mammography apparatus.

### Embodiment 2

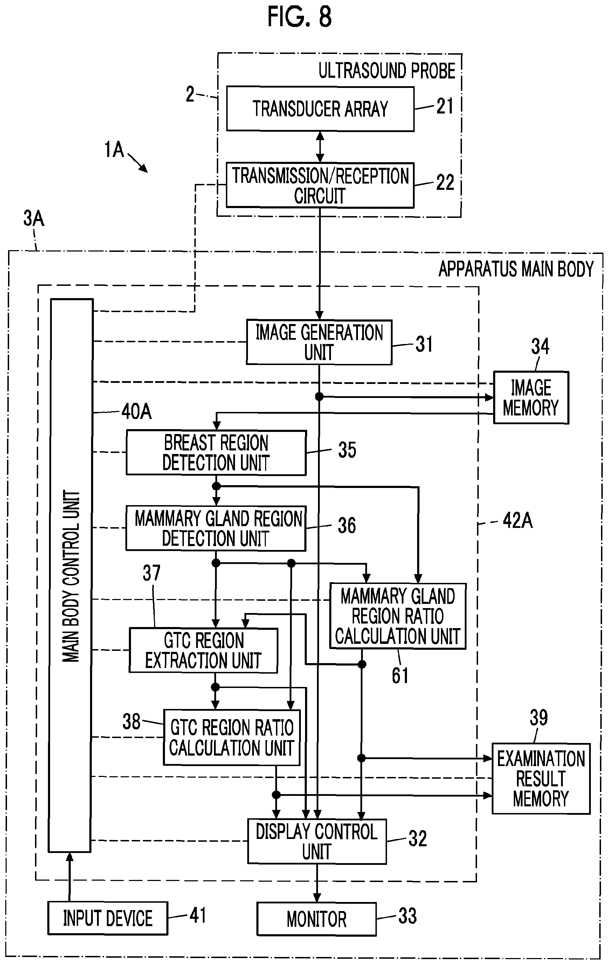

Fig. 8 illustrates a configuration of an ultrasound diagnostic apparatus 1A according to an embodiment 2. In the ultrasound diagnostic apparatus 1A, an ultrasound probe 2 is connected to an apparatus main body 3A. The apparatus main body 3A is obtained by newly adding a mammary gland region ratio calculation unit 61 to the apparatus main body 3 of the ultrasound diagnostic apparatus 1 according to the embodiment 1 illustrated in Fig. 1 and using a main body control unit 40A instead of the main body control unit 40. Other configurations are the same as those of the apparatus main body 3 according to the embodiment 1.

The ultrasound diagnostic apparatus 1A according to the embodiment 2 is configured to calculate not only the GTC region ratio of the GTC region R1 to the mammary gland region M but also a ratio of the mammary gland region M to the breast region BR and to display the calculated ratios on the monitor 33.

The breast region detection unit 35 and the mammary gland region detection unit 36 are connected to the mammary gland region ratio calculation unit 61, and the mammary gland region ratio calculation unit 61 is connected to the display control unit 32, the GTC region extraction unit 37, and the examination result memory 39.

A main body control unit 40A is connected to the image generation unit 31, the display control unit 32, the image memory 34, the breast region detection unit 35, the mammary gland region detection unit 36, the GTC region extraction unit 37, the GTC region ratio calculation unit 38, the examination result memory 39, and the mammary gland region ratio calculation unit 61. An input device 41 is connected to the main body control unit 40A.

A processor 42A is configured by the image generation unit 31, the display control unit 32, the breast region detection unit 35, the mammary gland region detection unit 36, the GTC region extraction unit 37, the GTC region ratio calculation unit 38, the main body control unit 40A, and the mammary gland region ratio calculation unit 61.

The mammary gland region ratio calculation unit 61 receives the detection result of the breast region BR by the breast region detection unit 35 and the detection result of the mammary gland region M by the mammary gland region detection unit 36, and calculates a ratio of the mammary gland region M to the breast region BR.

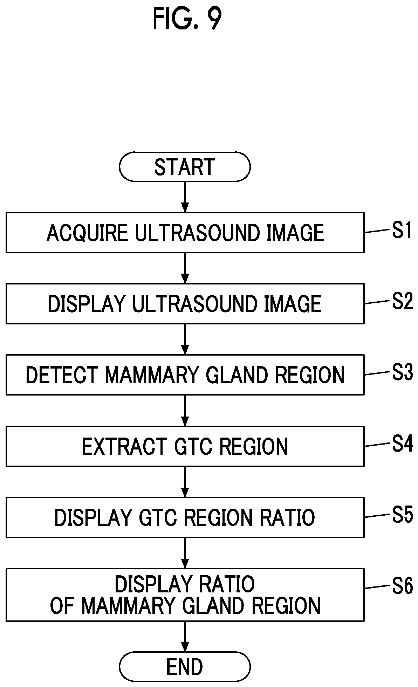

An operation of the ultrasound diagnostic apparatus 1A according to the embodiment 2 will be described with reference to a flowchart illustrated in Fig. 9.

Processing of step S1 to step S5 is the same as processing of step S1 to step S5 of the flowchart according to the embodiment 1 illustrated in Fig. 7. That is, in step S 1, an ultrasound image is acquired by imaging the breast of the subject, and in step S2, the ultrasound image is displayed on the monitor 33. In step S3, the breast region BR is detected from the ultrasound image by the breast region detection unit 35, and the mammary gland region M is detected by the mammary gland region detection unit 36. In step S4, the GTC region R1 is extracted from the mammary gland region M by the GTC region extraction unit 37. In step S5, the GTC region ratio of the GTC region R1 to the mammary gland region M is calculated by the GTC region ratio calculation unit 38, and the calculated GTC region ratio is displayed on the monitor 33.

Further, in the embodiment 2, subsequently, in step S6, a ratio of the mammary gland region M to the breast region BR is calculated by the mammary gland region ratio calculation unit 61 based on the breast region BR detected by the breast region detection unit 35 and the mammary gland region M detected by the mammary gland region detection unit 36, and the calculated ratio is displayed on the monitor 33 via the display control unit 32 and is stored in the examination result memory 39, as the examination result.

The ratio of the mammary gland region M to the breast region BR is known as a cancer risk factor, and can be measured by a mammography apparatus. On the other hand, according to the ultrasound diagnostic apparatus 1A of the embodiment 2, the mammary gland region ratio calculation unit 61 can calculate the ratio of the mammary gland region M to the breast region BR based on the ultrasound image generated by the image generation unit 31 without using a mammography apparatus.

Therefore, it is possible to estimate a cancer risk of the mammary gland region M of the subject with high accuracy, based on both the GTC region ratio that is calculated by the GTC region ratio calculation unit 38 and the ratio of the mammary gland region M to the breast region BR that is calculated by the mammary gland region ratio calculation unit 61.

In a case where the mammary glands retract and the volume of the breast decreases, fat regions increase on a side shallower than the front boundary line L1 illustrated in Fig. 4 and a side deeper than the rear boundary line L2 illustrated in Fig. 4. Further, in a case where fat regions occur between lobes in the mammary gland, the ratio of the mammary gland region M to the breast region BR may decrease. In a case where the ratio of the mammary gland region M to the breast region BR decreases, the cancer risk is also decreased. Thus, the GTC region extraction unit 37 may be configured to extract the GTC region R1 in the mammary gland region M only in a case where the ratio of the mammary gland region M detected by the mammary gland region ratio calculation unit 61 is higher than a set value.

That is, in a case where the ratio of the mammary gland region M detected by the mammary gland region ratio calculation unit 61 is higher than the set value, the GTC region R1 is extracted from the mammary gland region M by the GTC region extraction unit 37, and the GTC region ratio is calculated by the GTC region ratio calculation unit 38. The calculated GTC region ratio is displayed on the monitor 33. On the other hand, in a case where the ratio of the mammary gland region M detected by the mammary gland region ratio calculation unit 61 is equal to or lower than the set value, extraction of the GTC region R1 is not performed by the GTC region extraction unit 37, and thus calculation of the GTC region ratio is not also performed by the GTC region ratio calculation unit 38.

### Embodiment 3

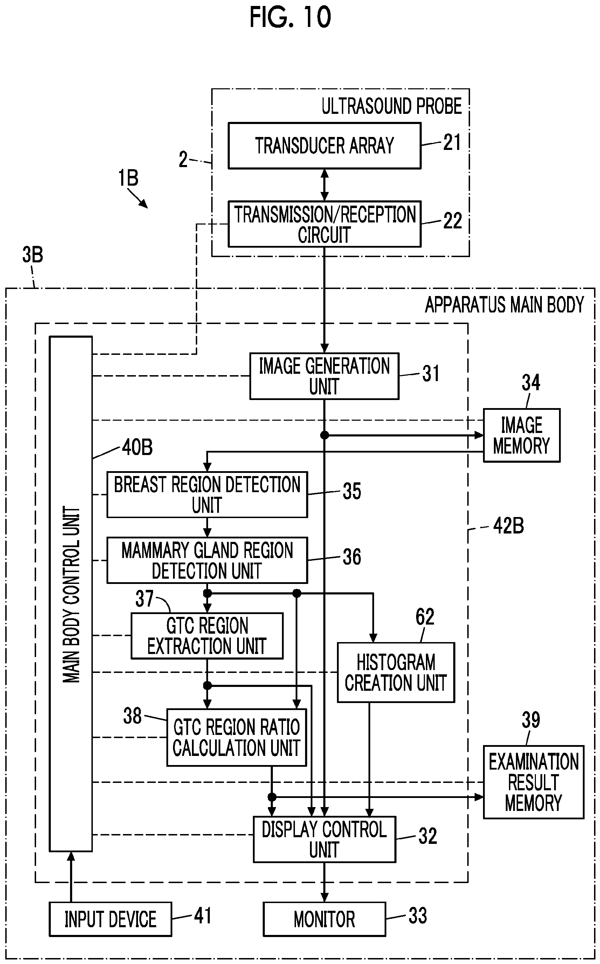

Fig. 10 illustrates a configuration of an ultrasound diagnostic apparatus 1B according to an embodiment 3. In the ultrasound diagnostic apparatus 1B, an ultrasound probe 2 is connected to an apparatus main body 3B. The apparatus main body 3B is obtained by newly adding a histogram creation unit 62 to the apparatus main body 3 of the ultrasound diagnostic apparatus 1 according to the embodiment 1 illustrated in Fig. 1 and using a main body control unit 40B instead of the main body control unit 40. Other configurations are the same as those of the apparatus main body 3 according to the embodiment 1.

The ultrasound diagnostic apparatus 1B according to the embodiment 3 is configured to allow the user to input the brightness threshold value Th which is used in a case where the GTC region extraction unit 37 creates a binarized image.

The mammary gland region detection unit 36 is connected to the histogram creation unit 62, and the histogram creation unit 62 is also connected to the display control unit 32.

A main body control unit 40B is connected to the image generation unit 31, the display control unit 32, the image memory 34, the breast region detection unit 35, the mammary gland region detection unit 36, the GTC region extraction unit 37, the GTC region ratio calculation unit 38, the examination result memory 39, and the histogram creation unit 62. An input device 41 is connected to the main body control unit 40B.

A processor 42B is configured by the image generation unit 31, the display control unit 32, the breast region detection unit 35, the mammary gland region detection unit 36, the GTC region extraction unit 37, the GTC region ratio calculation unit 38, the main body control unit 40B, and the histogram creation unit 62.

The histogram creation unit 62 creates a histogram of brightness of the mammary gland region M detected from the ultrasound image by the mammary gland region detection unit 36, and displays the created histogram on the monitor 33 via the display control unit 32.

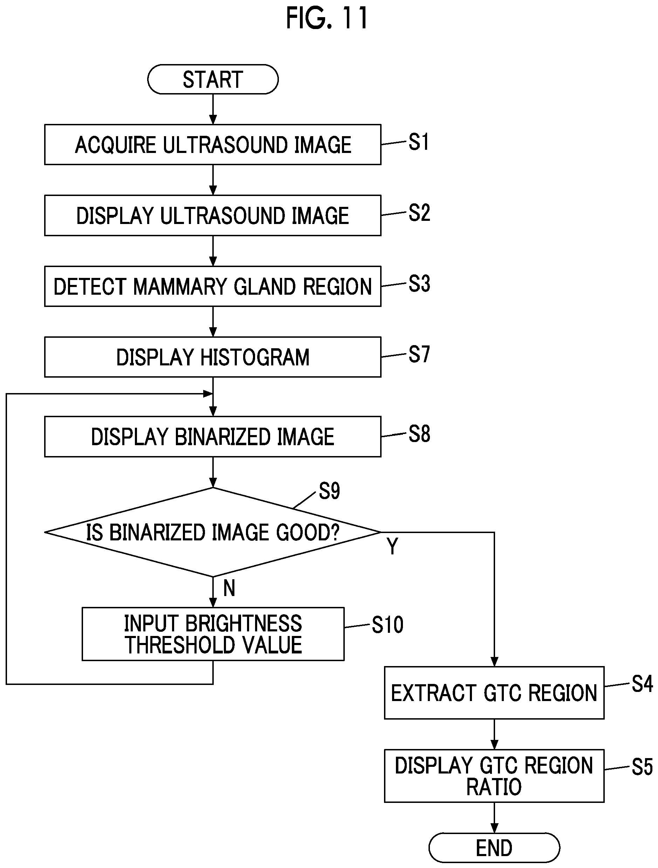

An operation of the ultrasound diagnostic apparatus 1B according to the embodiment 3 will be described with reference to a flowchart illustrated in Fig. 11.

Processing of step S1 to step S3 is the same as processing of step S1 to step S3 of the flowchart according to the embodiment 1 illustrated in Fig. 7. That is, in step S1, an ultrasound image is acquired by imaging the breast of the subject, and in step S2, the ultrasound image is displayed on the monitor 33. In step S3, the breast region BR is detected from the ultrasound image by the breast region detection unit 35, and the mammary gland region M is detected by the mammary gland region detection unit 36.

In the embodiment 3, subsequently, in step S7, a histogram of brightness of the mammary gland region M in the ultrasound image is created by the histogram creation unit 62, and the created histogram is displayed on the monitor 33 via the display control unit 32.

Further, in step S8, the mammary gland region M is binarized by the GTC region extraction unit 37 by using an initial value of the brightness threshold value Th, and the binarized image as illustrated in Fig. 6 is displayed on the monitor 33 via the display control unit 32.

In this way, the histogram created by the histogram creation unit 62, the binarized image created by the GTC region extraction unit 37, and the ultrasound image generated by the image generation unit 31 are displayed on the monitor 33.

Next, in step S9, whether or not the binarized image is good is determined by the user's visual comparison of the binarized image displayed on the monitor 33 with the ultrasound image. Specifically, it is determined whether or not the binarized image created by the GTC region extraction unit 37 matches with the ultrasound image generated by the image generation unit 31.

For example, in the binarized image as illustrated in Fig. 6, in a case where it is determined by the user that a black portion P1 properly represents the GTC region R1 appearing in the mammary gland region M of the ultrasound image as illustrated in Fig. 5 in the size and the shape, it is determined that the binarized image is good. On the other hand, in a case where it is determined by the user that a black portion P1 of the binarized image is quite different from the GTC region R1 appearing in the mammary gland region M of the ultrasound image in the size and the shape, it is determined that the binarized image is not good.

In a case where it is determined in step S9 that the binarized image is not good, the processing proceeds to step S10, and a new brightness threshold value Th is input by the user via the input device 41 based on the histogram, the binarized image, and the ultrasound image displayed on the monitor 33.

Thereafter, returning to step S8, a binarized image is created by using the new brightness threshold value Th which is input in step S10 by the GTC region extraction unit 37, and the binarized image is displayed on the monitor 33. Further, in step S9, whether or not the binarized image is good is determined again by the user.

In this way, processing of step S8 to step S10 is repeated until it is determined in step S9 that the binarized image is good.

In addition, in a case where it is determined in step S9 that the binarized image is good, the processing proceeds to step S4, and processing of step S4 and processing of step S5 are sequentially executed. The processing of step S4 and the processing of step S5 are the same as processing of step S4 and processing of step S5 in the flowchart according to the embodiment 1 illustrated in Fig. 7. That is, in step S4, the GTC region R1 is extracted from the mammary gland region M by using the binarized image by the GTC region extraction unit 37. In step S5, the GTC region ratio of the GTC region R1 to the mammary gland region M is calculated by the GTC region ratio calculation unit 38, and the GTC region ratio is displayed on the monitor 33.

As in the embodiment 3 described above, the histogram of brightness of the mammary gland region M that is created by the histogram creation unit 62 is displayed on the monitor 33, and a new brightness threshold value Th is input by the user based on the histogram, the binarized image, and the ultrasound image displayed on the monitor 33. Thereby, the GTC region extraction unit 37 can more accurately extract the GTC region R1 from the mammary gland region M. Therefore, it is possible to estimate a cancer risk of the mammary gland region M of the subject with high accuracy.

### Embodiment 4

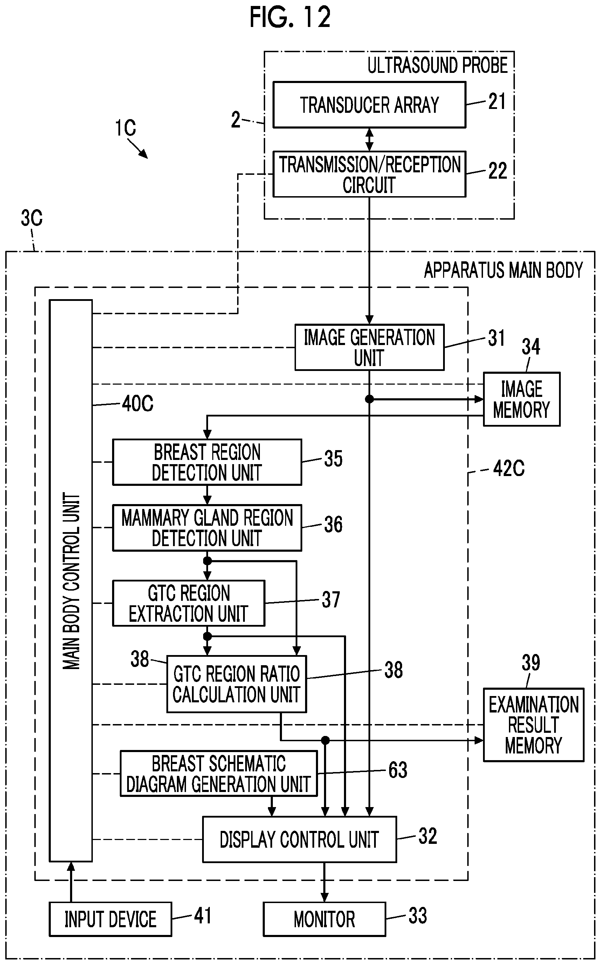

Fig. 12 illustrates a configuration of an ultrasound diagnostic apparatus 1C according to an embodiment 4. In the ultrasound diagnostic apparatus 1C, an ultrasound probe 2 is connected to an apparatus main body 3C. The apparatus main body 3C is obtained by newly adding a breast schematic diagram generation unit 63 to the apparatus main body 3 of the ultrasound diagnostic apparatus 1 according to the embodiment 1 illustrated in Fig. 1 and using a main body control unit 40C instead of the main body control unit 40. Other configurations are the same as those of the apparatus main body 3 according to the embodiment 1.

The ultrasound diagnostic apparatus 1C according to the embodiment 4 is configured to perform imaging of a plurality of ultrasound images at a plurality of predetermined locations of the breast and to calculate the GTC region ratio based on the plurality of ultrasound images.

The breast schematic diagram generation unit 63 is connected to the display control unit 32.

A main body control unit 40C is connected to the image generation unit 31, the display control unit 32, the image memory 34, the breast region detection unit 35, the mammary gland region detection unit 36, the GTC region extraction unit 37, the GTC region ratio calculation unit 38, the examination result memory 39, and the breast schematic diagram generation unit 63. An input device 41 is connected to the main body control unit 40C.

A processor 42C is configured by the image generation unit 31, the display control unit 32, the breast region detection unit 35, the mammary gland region detection unit 36, the GTC region extraction unit 37, the GTC region ratio calculation unit 38, the main body control unit 40C, and the breast schematic diagram generation unit 63.

In one ultrasound image obtained by bring the ultrasound probe 2 to come into contact with one location of the breast of the subject and imaging the breast of the subject, only a local tomographic plane of the breast can be seen. For this reason, in the ultrasound diagnostic apparatus 1C according to the embodiment 4, imaging of the ultrasound images is performed by using the ultrasound probe 2 at the plurality of predetermined locations of the breast.

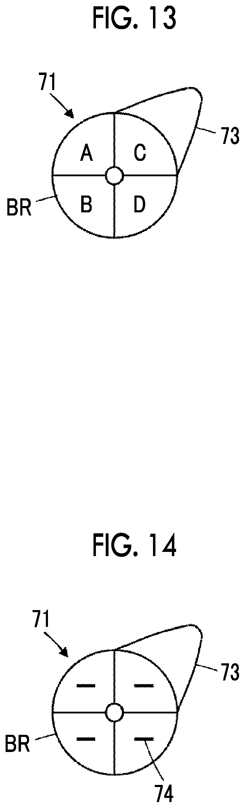

Therefore, the breast schematic diagram generation unit 63 generates, for example, a breast schematic diagram (also referred to as a schema or a body mark) 71 as illustrated in Fig. 13. The breast schematic diagram 71 illustrated in Fig. 13 schematically represents a left breast when viewed from the front, and includes a circle-shaped breast region BR and a substantially-triangle-shaped axillary region 73 representing an armpit and extending obliquely upward from the breast region BR. The breast region BR is divided into four regions including an inner upper region A, an inner lower region B, an outer upper region C, and an outer lower region D of the breast. The axillary region 73 is connected to a left oblique upper portion of the outer upper region C.

By horizontally inverting the breast schematic diagram 71 illustrated in Fig. 13, a breast schematic diagram schematically representing a right breast is obtained.

The breast schematic diagram generation unit 63 generates a breast schematic diagram 71 in which the plurality of predetermined locations for imaging the ultrasound images by bring the ultrasound probe 2 come to into contact with the breast are plotted as probe marks 74 as illustrated in Fig. 14, by using the inner upper region A, the inner lower region B, the outer upper region C, and the outer lower region D obtained by dividing the breast region BR into four. The generated breast schematic diagram 71 is displayed on the monitor 33.

In the breast schematic diagram 71 of Fig. 14, the probe marks 74 are plotted in all the four regions obtained by dividing the breast region BR. The probe mark 74 is represented by a line segment having a predetermined length, and not only indicates a position of each of the plurality of locations with which the ultrasound probe 2 is brought into contact but also indicates a direction of the ultrasound probe 2 which is brought into contact with each location by a direction of the line segment.

In the ultrasound diagnostic apparatus 1C according to the embodiment 4, the breast region detection unit 35 detects the breast region BR from each of the plurality of ultrasound images obtained by performing imaging at the plurality of locations defined by the breast schematic diagram 71. The mammary gland region detection unit 36 detects the mammary gland region M from the breast region BR of each of the plurality of ultrasound images. The GTC region extraction unit 37 extracts the GTC region R1 from the mammary gland region M of each of the plurality of ultrasound images. In addition, the GTC region ratio calculation unit 38 calculates a ratio of the GTC region R1 to the mammary gland region M in each of the plurality of ultrasound images. The calculated ratio is displayed on the monitor 33 via the display control unit 32.

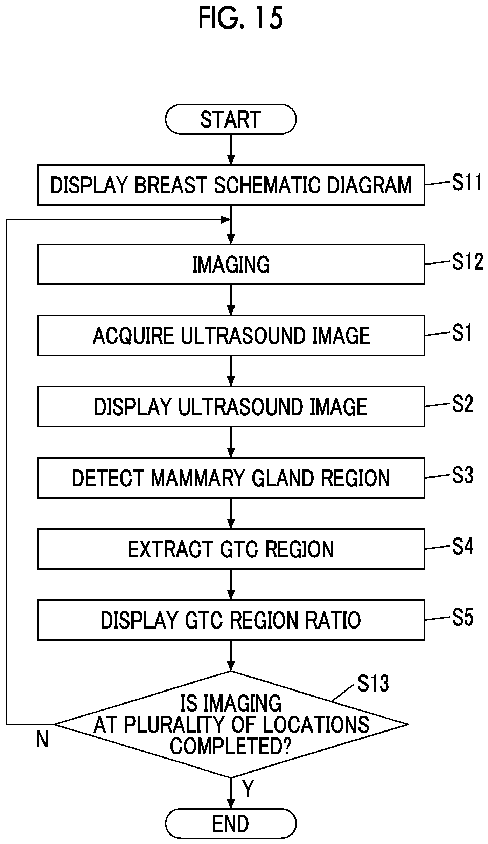

An operation of the ultrasound diagnostic apparatus 1C according to the embodiment 4 will be described with reference to a flowchart illustrated in Fig. 15.

First, in step S 11, a breast schematic diagram 71 as illustrated in Fig. 14 is generated by the breast schematic diagram generation unit 63, and the breast schematic diagram 71 is displayed on the monitor 33 via the display control unit 32. In the breast schematic diagram 71 illustrated in Fig. 14, the probe marks 74 are plotted in all the four regions obtained by dividing the breast region BR.

In step S12, the user confirms the breast schematic diagram 71 displayed on the monitor 33, and performs imaging of an ultrasound image according to the probe mark 74 plotted in one region of the four regions. That is, the ultrasound probe 2 is brought into contact with the breast of the subject in accordance with the position and the direction indicated by the probe mark 74. In this state, the plurality of transducers of the transducer array 21 start transmission and reception of ultrasound waves, and ultrasound echoes from the inside of the breast of the subject are received by the plurality of transducers of the transducer array 21.

Processing of step S1 to step S5 that is subsequent to step S12 is the same as processing of step S1 to step S5 of the flowchart according to the embodiment 1 illustrated in Fig. 7. In step S1, an ultrasound image is acquired, and in step S2, the ultrasound image is displayed on the monitor 33. In step S3, the mammary gland region M is detected from the ultrasound image. In step S4, the GTC region R1 is extracted from the mammary gland region M. In step S5, the GTC region ratio of the GTC region R1 to the mammary gland region M is calculated, and the calculated GTC region ratio is displayed on the monitor 33.

Thereafter, in step S13, it is determined whether or not imaging of ultrasound images at a plurality of predetermined locations is completed. Here, imaging according to a first probe mark 74 of the four probe marks 74 plotted on the breast schematic diagram 71 is completed, and imaging according to the remaining three probe marks 74 is not performed. Thus, it is determined that imaging at the plurality of locations is not yet completed, and the processing returns from step S13 to step S12.

In step S12, imaging according to a second probe mark 74 is performed. Subsequently, in step S1 to step S5, a GTC region ratio based on the ultrasound image acquired according to the second probe mark 74 is calculated, and the calculated GTC region ratio is displayed on the monitor 33. Thereafter, in step S13, it is determined whether or not imaging at the plurality of locations is completed.

Similarly, processing of step S12, step S1 to step S5, and step S13 is repeated until imaging of ultrasound images according to all the four probe marks 74 plotted on the breast schematic diagram 71 is completed.

In addition, in a case where it is determined in step S13 that imaging of ultrasound images according to all the four probe marks 74 is completed, a series of processing is completed.

Thereby, the GTC region ratio in each of four tomographic planes corresponding to the four probe marks 74 on the breast schematic diagram 71 is calculated, and the calculated GTC region ratio is displayed on the monitor 33.

By calculating the GTC region ratio based on the plurality of ultrasound images obtained by performing imaging at the plurality of locations, it is possible to improve accuracy of estimation of a cancer risk of the mammary gland region M of the subject, and perform diagnosis with higher reliability.

The GTC region ratio calculation unit 38 may display a plurality of GTC region ratios calculated at the plurality of predetermined locations on the monitor 33. Alternatively, the GTC region ratio calculation unit 38 may calculate an average value of a plurality of GTC region ratios at the plurality of locations and display the average value on the monitor 33 instead of the plurality of GTC region ratios or together with the plurality of GTC region ratios.

In the breast schematic diagram 71 illustrated in Fig. 14, the probe marks 74 are plotted in all the four regions obtained by dividing the breast region BR. On the other hand, the probe marks 74 may be plotted in two or more regions of the four regions instead of all the four regions. Further, the number of regions obtained by dividing the breast region BR is not limited to four. For example, the breast region BR may be divided into two regions or eight regions.

Further, instead of plotting the probe marks 74 in the regions obtained by dividing the breast region BR, as illustrated in Fig. 13, only regions such as the inner upper region A, the inner lower region B, the outer upper region C, and the outer lower region D are designated in the breast schematic diagram 71, and the user may perform imaging of an ultrasound image at a certain position in the designated region.

The number of divided regions in the breast schematic diagram 71, the number of the probe marks 74 to be plotted, and the like may be automatically set by the breast schematic diagram generation unit 63 under a control of the main body control unit 40C, or may be manually set by the user via the input device 41.

### Embodiment 5

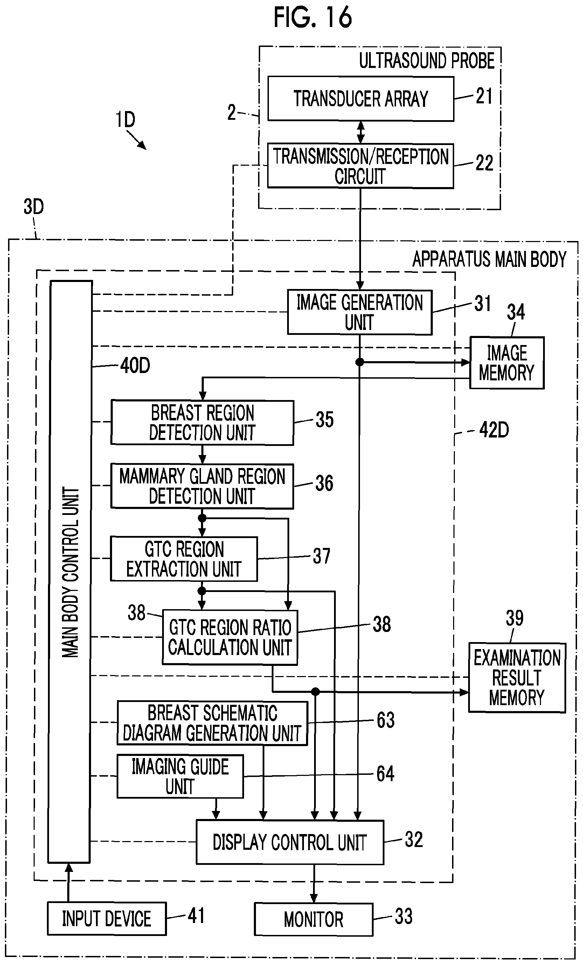

Fig. 16 illustrates a configuration of an ultrasound diagnostic apparatus 1D according to an embodiment 5. In the ultrasound diagnostic apparatus 1D, an ultrasound probe 2 is connected to an apparatus main body 3D. The apparatus main body 3D is obtained by newly adding an imaging guide unit 64 to the apparatus main body 3C of the ultrasound diagnostic apparatus 1C according to the embodiment 4 illustrated in Fig. 12 and using a main body control unit 40D instead of the main body control unit 40C. Other configurations are the same as those of the apparatus main body 3C according to the embodiment 4.

The ultrasound diagnostic apparatus 1D according to the embodiment 5 is configured to guide imaging of ultrasound images at a plurality of predetermined locations of the breast.

The imaging guide unit 64 is connected to the display control unit 32.

A main body control unit 40D is connected to the image generation unit 31, the display control unit 32, the image memory 34, the breast region detection unit 35, the mammary gland region detection unit 36, the GTC region extraction unit 37, the GTC region ratio calculation unit 38, the examination result memory 39, the breast schematic diagram generation unit 63, and the imaging guide unit 64. An input device 41 is connected to the main body control unit 40D.

A processor 42D is configured by the image generation unit 31, the display control unit 32, the breast region detection unit 35, the mammary gland region detection unit 36, the GTC region extraction unit 37, the GTC region ratio calculation unit 38, the main body control unit 40D, the breast schematic diagram generation unit 63, and the imaging guide unit 64.

The imaging guide unit 64 guides imaging of ultrasound images at a plurality of locations indicated by the probe marks 74 in the breast schematic diagram 71.

In the embodiment 5, an order of imaging at a plurality of predetermined locations is set in advance by, for example, the user. The breast schematic diagram generation unit 63 generates a breast schematic diagram 71 in which a probe mark 74 indicating a location at which next imaging is to be performed among the plurality of predetermined locations is plotted, and the breast schematic diagram 71 is displayed on the monitor 33.

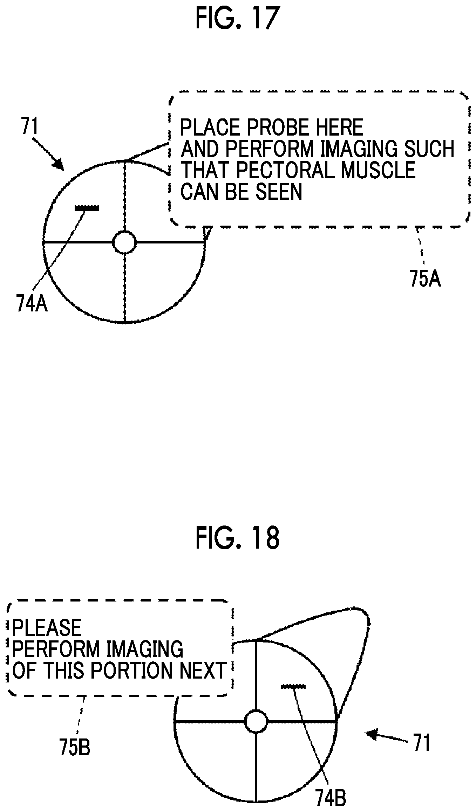

As illustrated in Fig. 17, the imaging guide unit 64 creates a guide 75A which corresponds to one probe mark 74A plotted in the breast schematic diagram 71 and includes a text, for example, "Place the probe here and perform imaging such that the pectoralis major muscle can be seen", and displays the guide 75A on the monitor 33.

In a case where imaging according to the probe mark 74A is completed, as illustrated in Fig. 18, the breast schematic diagram generation unit 63 displays, on the monitor 33, the breast schematic diagram 71 in which a probe mark 74B indicating a location at which next imaging is to be performed is plotted, and the imaging guide unit 64 creates a guide 75B which corresponds to the probe mark 74B and includes a text, for example, "Please perform imaging of this portion next" and displays the guide 75B on the monitor 33.

In this way, the imaging guide unit 64 guides imaging of ultrasound images corresponding to the probe marks 74 in the breast schematic diagram 71 displayed on the monitor 33.

As illustrated in Fig. 17 and Fig. 18, in a case where the guides 75A and 75B are positioned so as not to overlap the probe marks 74A and 74B plotted on the breast schematic diagram 71, the guides 75A and 75B can be displayed by being superimposed on a part of the breast schematic diagram 71. Alternatively, the guides 75A and 75B may be displayed at a position separated from the breast schematic diagram 71.

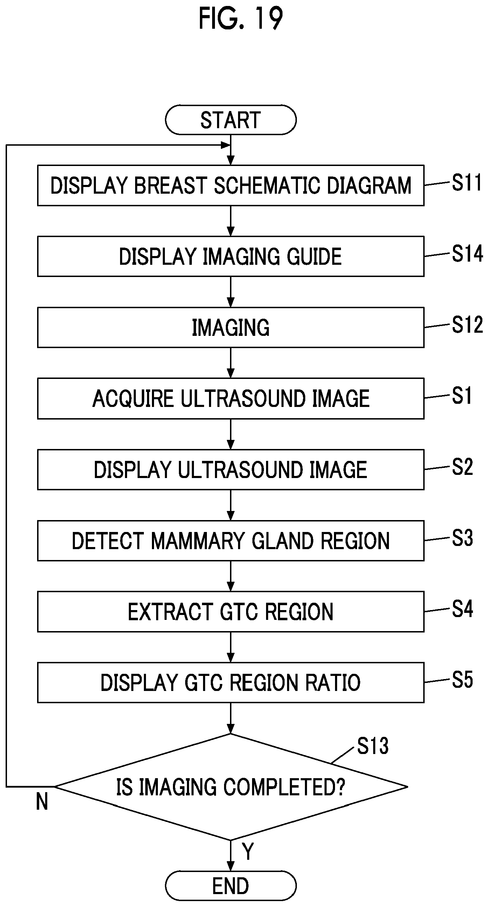

An operation of the ultrasound diagnostic apparatus 1D according to the embodiment 5 will be described with reference to a flowchart illustrated in Fig. 19.

First, in step S11, as illustrated in Fig. 17, a breast schematic diagram 71 in which one probe mark 74A indicating a location at which first imaging is to be performed among the plurality of predetermined locations is plotted is displayed on the monitor 33 by the breast schematic diagram generation unit 63. Further, in step S14, a guide 75A corresponding to the probe mark 74A is displayed on the monitor 33 by the imaging guide unit 64.

Subsequent processing of step S12 and step S1 to step S5 is the same as processing of step S12 and step S1 to step S5 of the flowchart according to the embodiment 4 illustrated in Fig. 15. Imaging of an ultrasound image is performed according to the probe mark 74A of the breast schematic diagram 71. Further, a GTC region ratio of the GTC region R1 to the mammary gland region M is calculated, and the GTC region ratio is displayed on the monitor 33.

In step S5, the GTC region ratio calculation unit 38 may display a plurality of GTC region ratios calculated at the plurality of predetermined locations on the monitor 33. Alternatively, the GTC region ratio calculation unit 38 may calculate an average value of a plurality of GTC region ratios at the plurality of locations and display the average value on the monitor 33 instead of the plurality of GTC region ratios or together with the plurality of GTC region ratios.

Thereafter, in step S13, as in the embodiment 4, it is determined whether or not imaging of ultrasound images at a plurality of predetermined locations is completed. Here, since only the first imaging is completed according to the probe mark 74A, the processing returns from step S13 to step S 11.

In step S11, for example, as illustrated in Fig. 18, a breast schematic diagram 71 in which a probe mark 74B indicating a location at which second imaging is to be performed among the plurality of predetermined locations is plotted is displayed on the monitor 33 by the breast schematic diagram generation unit 63. In subsequent step S14, a guide 75B corresponding to the probe mark 74B is displayed on the monitor 33 by the imaging guide unit 64.

In subsequent step S12 and step S1 to step S5, the imaging at the second location is performed according to the probe mark 74B. In addition, a GTC region ratio of the GTC region R1 to the mammary gland region M is calculated, and the calculated GTC region ratio is displayed on the monitor 33. Further, in step S13, it is determined whether or not imaging at the plurality of locations is completed.

In this way, processing of step S11, step S14, step S12, step S1 to step S5, and step S13 is repeated until all imaging at the plurality of predetermined locations is completed.

Further, in step S13, in a case where it is determined that all imaging at the plurality of predetermined locations is completed, a series of processing is completed.

In this way, the breast schematic diagram generation unit 63 displays, on the monitor 33, the breast schematic diagram 71 in which the probe mark 74 indicating a location for next imaging is plotted, and the imaging guide unit 64 displays, on the monitor 33, the guide 75B corresponding to the probe mark 74 indicating a location for next imaging. Thereby, even in a case of a user who is not skilled in imaging of ultrasound images of a breast, it is possible to accurately and quickly perform imaging and to display the GTC region ratio on the monitor 33.

In the embodiment 5, the apparatus main body 3D may include a speaker (not illustrated), and the imaging guide unit 64 may provide a voice guide for imaging at the plurality of predetermined locations via the speaker.

The method of connecting the ultrasound probe 2 and the apparatus main body 3 in the embodiments 1 to 5 is not particularly limited, and a wired connection method or a wireless connection method may be used.

In the embodiments 1 to 5, the ultrasound probe 2 includes the transmission/reception circuit 22. On the other hand, the apparatus main body 3 may include the transmission/reception circuit 22. In addition, the apparatus main body 3 includes the image generation unit 31. On the other hand, the ultrasound probe 2 may include the image generation unit 31. Further, the image generation unit 31 illustrated in Fig. 3 includes the signal processing unit 51, the DSC 52, and the image processing unit 53. On the other hand, the ultrasound probe 2 may include only the signal processing unit 51, and the apparatus main body 3 may include the DSC 52 and the image processing unit 53.

In addition, in the embodiments 1 to 5, as the apparatus main body 3, a portable or handheld compact apparatus main body can be used, and a stationary apparatus main body can also be used.

### Explanation of References

1, 1A, 1B, 1C, 1D: ultrasound diagnostic apparatus
2: ultrasound probe
3, 3A, 3B, 3C, 3D: apparatus main body
4: ultrasound image analysis apparatus
21: transducer array
22: transmission/reception circuit
23: pulser
24: amplification unit
25: AD conversion unit
26: beam former
31: image generation unit
32: display control unit
33: monitor
34: image memory
35: breast region detection unit
36: mammary gland region detection unit
37: GTC region extraction unit
38: GTC region ratio calculation unit
39: examination result memory
40, 40A, 40B, 40C, 40D: main body control unit
41: input device
42, 42A, 42B, 42C, 42D: processor
51: signal processing unit
52: DSC
53: image processing unit
61: mammary gland region ratio calculation unit
62: histogram creation unit
63: breast schematic diagram generation unit
64: imaging guide unit
71: breast schematic diagram
73: axillary region
74, 74A, 74B: probe mark
75A, 75B: guide
S: skin
BR: breast region
M: mammary gland region
L 1: front boundary line
L2: rear boundary line
T: pectoralis major muscle
R1: GTC region
R2: edematous region
P1: black portion
P2: white portion

## Claims

1. An ultrasound image analysis apparatus (4) comprising:
a mammary gland region detection unit (36) that detects a mammary gland region from an ultrasound image obtained by imaging a breast of a subject;
a glandular tissue component region extraction unit (37) that extracts a glandular tissue component region including mammary ducts, lobules, and peripheral stromata in the mammary gland region detected by the mammary gland region detection unit; and
a glandular tissue component region ratio calculation unit (38) that calculates a ratio of the glandular tissue component region to the mammary gland region.

2. The ultrasound image analysis apparatus according to claim 1,
wherein the mammary gland region detection unit (36) detects the mammary gland region by image recognition of the ultrasound image.

3. The ultrasound image analysis apparatus according to claim 2, further comprising:
a breast region detection unit (35) that detects a breast region located between a skin and a pectoralis major muscle from the ultrasound image,
wherein the mammary gland region detection unit (36) recognizes a front boundary line and a rear boundary line in the breast region detected by the breast region detection unit, and detects a region between the front boundary line and the rear boundary line, as the mammary gland region.

4. The ultrasound image analysis apparatus according to claim 1,
wherein the mammary gland region detection unit (36) detects the mammary gland region from the ultrasound image by using deep learning.

5. The ultrasound image analysis apparatus according to any one of claims 1 to 4,
wherein the glandular tissue component region extraction unit (37) extracts the glandular tissue component region by binarizing the mammary gland region of the ultrasound image by using a brightness threshold value.

6. The ultrasound image analysis apparatus according to any one of claims 1 to 4,
wherein the glandular tissue component region extraction unit (37) extracts the glandular tissue component region from the mammary gland region of the ultrasound image by using deep learning.

7. The ultrasound image analysis apparatus according to any one of claims 1 to 6,
wherein the glandular tissue component region ratio calculation unit (38) calculates the ratio of the glandular tissue component region to the mammary gland region based on the number of occupied pixels of the mammary gland region and the number of occupied pixels of the glandular tissue component region in the ultrasound image.

8. The ultrasound image analysis apparatus according to any one of claims 1 to 7,
wherein the mammary gland region detection unit (36) detects the mammary gland region from each of a plurality of the ultrasound images obtained by imaging the breast of the subject at a plurality of predetermined locations of the breast of the subject,
the glandular tissue component region extraction unit (37) extracts the glandular tissue component region from each of the mammary gland regions of the plurality of ultrasound images, and
the glandular tissue component region ratio calculation unit (38) calculates the ratio of the glandular tissue component region to the mammary gland region in each of the plurality of ultrasound images.

9. The ultrasound image analysis apparatus according to claim 8,
wherein the glandular tissue component region ratio calculation unit (38) calculates an average value of ratios of the glandular tissue component regions to the mammary gland regions in the plurality of ultrasound images.

10. The ultrasound image analysis apparatus according to any one of claims 1 to 9,
wherein the ultrasound image is a three-dimensional ultrasound image, and
the glandular tissue component region ratio calculation unit (38) calculates the ratio of the glandular tissue component region to the mammary gland region based on a volume of the mammary gland region detected by the mammary gland region detection unit (36) and a volume of the glandular tissue component region extracted by the glandular tissue component region extraction unit (37).

11. An ultrasound diagnostic apparatus comprising:
a monitor (33) that displays an ultrasound image obtained by imaging a breast of a subject; and
the ultrasound image analysis apparatus (4) according to any one of claims 1 to 10,
wherein the mammary gland region detection unit (36) detects the mammary gland region from the ultrasound image, and
the glandular tissue component region ratio calculation unit (38) displays the calculated ratio of the glandular tissue component region to the mammary gland region on the monitor.

12. The ultrasound diagnostic apparatus according to claim 11, further comprising:
a mammary gland region ratio calculation unit (61) that calculates a ratio of the mammary gland region to the breast region and displays the calculated ratio on the monitor.

13. The ultrasound diagnostic apparatus according to claim 12,
wherein the glandular tissue component region extraction unit (37) extracts the glandular tissue component region in the mammary gland region only in a case where the ratio of the mammary gland region calculated by the mammary gland region ratio calculation unit is higher than a set value.

14. The ultrasound diagnostic apparatus according to claim 13,
wherein the glandular tissue component region extraction unit (37) extracts the glandular tissue component region by binarizing the mammary gland region of the ultrasound image by using a brightness threshold value, and displays a binarized image obtained by binarizing the mammary gland region on the monitor.

15. The ultrasound diagnostic apparatus according to claim 14,
wherein the brightness threshold value is a constant value.

16. The ultrasound diagnostic apparatus according to claim 13 or 14,
wherein the glandular tissue component region extraction unit (37) detects an edge of the glandular tissue component region in the ultrasound image, and automatically sets the brightness threshold value based on a change in brightness value at the detected edge.

17. The ultrasound diagnostic apparatus according to claim 14, further comprising:
a histogram creation unit (62) that creates a histogram of brightness of the mammary gland region in the ultrasound image and displays the created histogram on the monitor,
wherein the brightness threshold value is set by a user based on the histogram, the binarized image, and the ultrasound image which are displayed on the monitor.

18. The ultrasound diagnostic apparatus according to any one of claims 11 to 17, further comprising:
a breast schematic diagram generation unit (63) that generates a schematic diagram of the breast on which a plurality of predetermined locations are plotted and displays the schematic diagram on the monitor.

19. The ultrasound diagnostic apparatus according to claim 18, further comprising:
an imaging guide unit (64) that guides a user to perform imaging of the ultrasound images at the plurality of predetermined locations.

20. The ultrasound diagnostic apparatus according to any one of claims 11 to 19,
wherein the glandular tissue component region ratio calculation unit (38) stores the calculated ratio of the glandular tissue component region in a tag associated with the ultrasound image.

21. The ultrasound diagnostic apparatus according to any one of claims 11 to 20,
wherein the glandular tissue component region ratio calculation unit (38) displays, on the monitor (33), a past ratio of the glandular tissue component region that is calculated based on the past ultrasound image of the subject, together with a latest ratio of the glandular tissue component region that is calculated based on the latest ultrasound image of the subject.

22. The ultrasound diagnostic apparatus according to any one of claims 11 to 21, further comprising:
an ultrasound probe (2); and
an image generation unit (31) that generates the ultrasound image obtained by imaging the breast of the subject by transmitting and receiving an ultrasound beam to and from the subject using the ultrasound probe.

23. A computer-implemented control method for an ultrasound image analysis apparatus, the method comprising
detecting a mammary gland region from an ultrasound image obtained by imaging a breast of a subject (S3);
extracting a glandular tissue component region including mammary ducts, lobules, and peripheral stromata in the mammary gland region (S4); and
calculating a ratio of the glandular tissue component region to the mammary gland region.

## Patentansprüche

1. Ultraschallbildanalysegerät (4), aufweisend:
eine Brustdrüsenbereichs-Erkennungseinheit (36), die einen Brustdrüsenbereich aus einem Ultraschallbild erkennt, das durch Abbilden einer Brust einer Person erhalten wird;
eine Drüsengewebekomponentenbereichs-Extraktionseinheit (37), die einen Drüsengewebekomponentenbereich extrahiert, der Brustdrüsengänge, Läppchen und periphere Stromata in dem von der Brustdrüsenbereichs-Erkennungseinheit erfassten Brustdrüsenbereich enthält; und
eine Drüsengewebekomponentenbereichsverhältnis-Berechnungseinheit (38), die ein Verhältnis zwischen dem Drüsengewebekomponentenbereich und der Brustdrüsenregion errechnet.

2. Ultraschallbildanalysegerät nach Anspruch 1,
wobei die Brustdrüsenbereichs-Erkennungseinheit den Brustdrüsenbereich durch Bilderkennung des Ultraschallbildes erkennt.

3. Ultraschallbildanalysegerät nach Anspruch 2, das außerdem Folgendes aufweist:
eine Brustbereichs-Erkennungseinheit (35), die einen Brustbereich, der sich zwischen einer Haut und einem großen Brustmuskel befindet, anhand des Ultraschallbildes erfasst,
wobei die Brustdrüsenbereichs-Erkennungseinheit (36) eine vordere Grenzlinie und eine hintere Grenzlinie in dem von der Brustbereichs-Erkennungseinheit erfassten Brustbereich erfasst, und einen Bereich zwischen der vorderen Grenzlinie und der hinteren Grenzlinie als den Brustdrüsenbereich erkennt.

4. Ultraschallbildanalysegerät nach Anspruch 1,
wobei die Brustdrüsenbereichs-Erkennungseinheit (36) den Brustdrüsenbereich aus dem Ultraschallbild durch Verwendung von Deep Learning erkennt.

5. Ultraschallbildanalysegerät nach einem der Ansprüche 1 bis 4,
wobei die Drüsengewebekomponentenbereichs-Extraktionseinheit (37) den Drüsengewebekomponentenbereich durch Binarisierung des Brustdrüsenbereichs des Ultraschallbildes unter Verwendung eines Helligkeitsschwellenwerts extrahiert.

6. Ultraschallbildanalysegerät nach einem der Ansprüche 1 bis 4,
wobei die Drüsengewebekomponentenbereichs-Extraktionseinheit (37) den Drüsengewebekomponentenbereich aus dem Brustdrüsenbereich des Ultraschallbildes durch Verwendung von Deep Learning extrahiert.

7. Ultraschallbildanalysegerät nach einem der Ansprüche 1 bis 6,
wobei die Drüsengewebekomponentenbereichsverhältnis-Berechnungseinheit (38) das Verhältnis des Drüsengewebekomponentenbereichs zu der Brustdrüsenregion auf der Grundlage der Anzahl der besetzten Pixel der Brustdrüsenregion und der Anzahl der besetzten Pixel des Drüsengewebekomponentenbereichs in dem Ultraschallbild berechnet.

8. Ultraschallbildanalysegerät nach einem der Ansprüche 1 bis 7,
wobei die Brustdrüsenbereichs-Erkennungseinheit (36) den Brustdrüsenbereich von jedem einer Vielzahl von Ultraschallbildern erfasst, die durch Abbilden der Brust des Patienten an einer Vielzahl von vorbestimmten Stellen der Brust des Patienten erhalten werden,
die Drüsengewebekomponentenbereichs-Extraktionseinheit (37) den Bereich der Drüsengewebekomponente aus jedem der Brustdrüsenbereiche der mehreren Ultraschallbilder extrahiert, und
die Drüsengewebekomponentenbereichsverhältnis-Berechnungseinheit (38) das Verhältnis des Drüsengewebekomponentenbereichs zu der Brustdrüsenregion in jedem der mehreren Ultraschallbilder berechnet.

9. Ultraschallbildanalysegerät nach Anspruch 8,
wobei die Drüsengewebekomponentenbereichsverhältnis-Berechnungseinheit (38) einen Durchschnittswert der Verhältnisse der Drüsengewebekomponentenbereiche zu den Brustdrüsenbereichen in der Vielzahl der Ultraschallbilder berechnet.

10. Ultraschallbildanalysegerät nach einem der Ansprüche 1 bis 9,
wobei das Ultraschallbild ein dreidimensionales Ultraschallbild ist, und
die Drüsengewebekomponentenbereichsverhältnis-Berechnungseinheit (38) das Verhältnis des Drüsengewebekomponentenbereichs zu der Brustdrüsenregion auf der Grundlage eines Volumens der Brustdrüsenregion, die von der Brustdrüsenbereichs-Erkennungseinheit (36) erfasst wurde, und eines Volumens der Drüsengewebekomponentenbereich, die von der Drüsengewebekomponentenbereichs-Extraktionseinheit (37) extrahiert wurde, berechnet.

11. Ultraschalldiagnosegerät, aufweisend:
einen Monitor (33), der ein Ultraschallbild anzeigt, das durch Abbildung der Brust einer Person erhalten wurde; und
das Ultraschallbildanalysegerät (4) nach einem der Ansprüche 1 bis 10, wobei die Brustdrüsenbereichs-Erkennungseinheit (36) den Brustdrüsenbereich aus dem Ultraschallbild erfasst, und
die Drüsengewebekomponentenbereichsverhältnis-Berechnungseinheit (38) das berechnete Verhältnis des Drüsengewebekomponentenbereichs zur Brustdrüsenregion auf dem Monitor anzeigt.

12. Ultraschalldiagnosegerät nach Anspruch 11, das außerdem Folgendes aufweist:
eine Brustdrüsenbereich-Brustbereich-Verhältnisberechnungseinheit (61), die ein Verhältnis zwischen dem Brustdrüsenbereich und dem Brustbereich berechnet und das berechnete Verhältnis auf dem Monitor anzeigt.

13. Ultraschalldiagnosegerät nach Anspruch 12,
wobei die Drüsengewebekomponentenbereichs-Extraktionseinheit (37) den Drüsengewebekomponentenbereich im Brustdrüsenbereich nur in dem Fall extrahiert, in dem das Verhältnis des Brustdrüsenbereichs, das von der Drüsengewebekomponentenbereichsverhältnis-Berechnungseinheit berechnet wird, höher als ein eingestellter Wert ist.

14. Ultraschalldiagnosegerät nach Anspruch 13,
wobei die Drüsengewebekomponentenbereichs-Extraktionseinheit (37) den Drüsengewebekomponentenbereich durch Binarisieren des Brustdrüsenbereichs des Ultraschallbildes unter Verwendung eines Helligkeitsschwellenwerts extrahiert und ein durch Binarisieren des Brustdrüsenbereichs erhaltenes binarisiertes Bild auf dem Monitor anzeigt.

15. Ultraschalldiagnosegerät nach Anspruch 14,
wobei der Helligkeitsschwellenwert ein konstanter Wert ist.

16. Ultraschalldiagnosegerät nach Anspruch 13 oder 14,
wobei die Drüsengewebekomponentenbereichs-Extraktionseinheit (37) eine Kante des Drüsengewebekomponentenbereichs in dem Ultraschallbild erfasst und den Helligkeitsschwellenwert automatisch auf der Grundlage einer Änderung des Helligkeitswertes an der erfassten Kante einstellt.

17. Ultraschalldiagnosegerät nach Anspruch 14, das außerdem aufweist:
eine Histogrammerzeugungseinheit (62), die ein Histogramm der Helligkeit des Brustdrüsenbereichs in dem Ultraschallbild erzeugt und das erzeugte Histogramm auf dem Monitor anzeigt,
wobei der Helligkeitsschwellenwert von einem Benutzer auf der Grundlage des Histogramms, des binarisierten Bildes und des Ultraschallbildes, die auf dem Monitor angezeigt werden, eingestellt wird.

18. Ultraschalldiagnosegerät nach einem der Ansprüche 11 bis 17, ferner aufweisend:
eine Schematisches-Brustdiagramm-Erzeugungseinheit (63), die ein schematisches Diagramm der Brust erzeugt, auf dem eine Vielzahl vorbestimmter Stellen eingezeichnet sind, und das schematische Diagramm auf dem Monitor anzeigt.

19. Ultraschalldiagnosegerät nach Anspruch 18, ferner aufweisend:
eine Abbildungsführungseinheit (64), die einen Benutzer anleitet, um die Abbildung der Ultraschallbilder an der Vielzahl der vorbestimmten Stellen durchzuführen.

20. Ultraschalldiagnosegerät nach einem der Ansprüche 11 bis 19,
wobei die Drüsengewebekomponentenbereichsverhältnis-Berechnungseinheit (38) das berechnete Verhältnis des Drüsengewebekomponentenbereichs in einer mit dem Ultraschallbild verbundenen Markierung speichert.

21. Ultraschalldiagnosegerät nach einem der Ansprüche 11 bis 20,
wobei die Drüsengewebekomponentenbereichsverhältnis-Berechnungseinheit (38) auf dem Monitor (33) ein vergangenes Verhältnis des Drüsengewebekomponentenbereichs, das auf der Grundlage des vergangenen Ultraschallbildes der Person berechnet wird, zusammen mit einem letzten Verhältnis des Drüsengewebekomponentenbereichs, das auf der Grundlage des letzten Ultraschallbildes der Person berechnet wird, anzeigt.

22. Ultraschalldiagnosegerät nach einem der Ansprüche 11 bis 21, ferner aufweisend:
eine Ultraschallsonde (2); und
eine Bilderzeugungseinheit (31), die das Ultraschallbild erzeugt, das durch Abbilden der Brust der Person durch Senden und Empfangen eines Ultraschallstrahls zu und von der Person unter Verwendung der Ultraschallsonde erhalten wird.

23. Computerimplementiertes Steuerungsverfahren für ein Ultraschallbildanalysegerät, wobei das Verfahren Folgendes umfasst:
Erkennen (S3) eines Brustdrüsenbereichs aus einem Ultraschallbild, das durch die Abbildung einer Brust einer Person erhalten wurde;
Extrahieren (S4) eines Drüsengewebekomponentenbereichs, der Brustdrüsengänge, Läppchen und periphere Stromata im Brustdrüsenbereich enthält; und
Berechnen des Verhältnisses zwischen dem Drüsengewebebereich und dem Brustdrüsenbereich.

## Revendications

1. Appareil d'analyse d'images ultrasonores (4) comprenant :
une unité de détection de la région de la glande mammaire (36) qui détecte une région de la glande mammaire à partir d'une image ultrasonore obtenue par imagerie d'un sein d'un sujet ;
une unité d'extraction de la région des composants du tissu glandulaire (37) qui extrait une région des composants du tissu glandulaire comprenant les canaux mammaires, les lobules et le stroma périphérique dans la région de la glande mammaire détectée par l'unité de détection de la région de la glande mammaire ; et
une unité de calcul du ratio de la région du composant du tissu glandulaire (38) qui calcule un ratio de la région du composant du tissu glandulaire par rapport à la région de la glande mammaire.

2. Appareil d'analyse d'images ultrasonores selon la revendication 1,
l'unité de détection de la région de la glande mammaire (36) détecte la région de la glande mammaire par reconnaissance d'image de l'image ultrasonore.

3. Appareil d'analyse d'images ultrasonores selon la revendication 2, comprenant en outre :
une unité de détection de la région mammaire (35) qui détecte une région mammaire située entre la peau et le muscle grand pectoral à partir de l'image ultrasonore,
l'unité de détection de la région de la glande mammaire (36) reconnaît une ligne de démarcation avant et une ligne de démarcation arrière dans la région du sein détectée par l'unité de détection de la région du sein, et détecte une région entre la ligne de démarcation avant et la ligne de démarcation arrière comme étant la région de la glande mammaire.

4. Appareil d'analyse d'images ultrasonores selon la revendication 1,
l'unité de détection de la région de la glande mammaire (36) détecte la région de la glande mammaire à partir de l'image ultrasonore en utilisant l'apprentissage profond.

5. Appareil d'analyse d'images ultrasonores selon l'une des revendications 1 à 4,
l'unité d'extraction de la région composant le tissu glandulaire (37) extrait la région composant le tissu glandulaire en binarisant la région de la glande mammaire de l'image ultrasonore à l'aide d'une valeur seuil de luminosité.

6. Appareil d'analyse d'images ultrasonores selon l'une des revendications 1 à 4,
l'unité d'extraction de la région des composants du tissu glandulaire (37) extrait la région des composants du tissu glandulaire de la région de la glande mammaire de l'image ultrasonore à l'aide de l'apprentissage profond.

7. Appareil d'analyse d'images ultrasonores selon l'une des revendications 1 à 6,
l'unité de calcul du rapport entre la région du tissu glandulaire et la région de la glande mammaire (38) calcule le rapport entre la région du tissu glandulaire et la région de la glande mammaire sur la base du nombre de pixels occupés de la région de la glande mammaire et du nombre de pixels occupés de la région du tissu glandulaire dans l'image ultrasonore.

8. Appareil d'analyse d'images ultrasonores selon l'une des revendications 1 à 7,
l'unité de détection de la région de la glande mammaire (36) détecte la région de la glande mammaire sur chacune des images ultrasonores obtenues par imagerie du sein du sujet à plusieurs endroits prédéterminés du sein du sujet,
l'unité d'extraction de la région composant le tissu glandulaire (37) extrait la région composant le tissu glandulaire de chacune des régions de la glande mammaire de la pluralité d'images ultrasonores, et
l'unité de calcul du rapport de la région du composant du tissu glandulaire (38) calcule le rapport de la région du composant du tissu glandulaire à la région de la glande mammaire dans chacune de la pluralité d'images ultrasonores.

9. Appareil d'analyse d'images ultrasonores selon la revendication 8,
l'unité de calcul du rapport des régions des composants du tissu glandulaire (38) calcule une valeur moyenne des rapports entre les régions des composants du tissu glandulaire et les régions de la glande mammaire dans la pluralité d'images ultrasonores.

10. Appareil d'analyse d'images ultrasonores selon l'une des revendications 1 à 9,
l'image ultrasonore est une image ultrasonore tridimensionnelle, et
l'unité de calcul du rapport de la région du composant du tissu glandulaire (38) calcule le rapport de la région du composant du tissu glandulaire à la région de la glande mammaire sur la base d'un volume de la région de la glande mammaire détecté par l'unité de détection de la région de la glande mammaire (36) et d'un volume de la région du composant du tissu glandulaire extrait par l'unité d'extraction de la région du composant du tissu glandulaire (37).

11. Appareil de diagnostic par ultrasons comprenant :
un moniteur (33) qui affiche une image ultrasonore obtenue par imagerie du sein d'un sujet ; et
l'appareil d'analyse d'images ultrasonores (4) selon l'une quelconque des revendications 1 à 10, dans lequel l'unité de détection de la région de la glande mammaire (36) détecte la région de la glande mammaire à partir de l'image ultrasonore, et
l'unité de calcul du rapport entre la région du tissu glandulaire et la région de la glande mammaire (38) affiche le rapport calculé entre la région du tissu glandulaire et la région de la glande mammaire sur le moniteur.

12. Appareil de diagnostic par ultrasons selon la revendication 11, comprenant en outre :
une unité de calcul du rapport de la région de la glande mammaire (61) qui calcule un rapport entre la région de la glande mammaire et la région du sein et affiche le rapport calculé sur le moniteur.

13. Appareil de diagnostic par ultrasons selon la revendication 12,
l'unité d'extraction de la région de composants du tissu glandulaire (37) extrait la région de composants du tissu glandulaire dans la région de la glande mammaire uniquement dans le cas où le rapport de la région de la glande mammaire calculé par l'unité de calcul du rapport de la région de la glande mammaire est supérieur à une valeur définie.

14. Appareil de diagnostic par ultrasons selon la revendication 13,
l'unité d'extraction de la région composant le tissu glandulaire (37) extrait la région composant le tissu glandulaire en binarisant la région de la glande mammaire de l'image ultrasonore à l'aide d'une valeur seuil de luminosité, et affiche une image binarisée obtenue par binarisation de la région de la glande mammaire sur le moniteur.

15. Appareil de diagnostic par ultrasons selon la revendication 14,
la valeur seuil de luminosité est une valeur constante.

16. Appareil de diagnostic par ultrasons selon la revendication 13 ou 14,
l'unité d'extraction de la région du tissu glandulaire (37) détecte un bord de la région du tissu glandulaire dans l'image ultrasonore et définit automatiquement la valeur seuil de luminosité sur la base d'un changement de la valeur de luminosité au niveau du bord détecté.

17. Appareil de diagnostic par ultrasons selon la revendication 14, comprenant en outre :
une unité de création d'histogramme (62) qui crée un histogramme de luminosité de la région de la glande mammaire dans l'image ultrasonore et affiche l'histogramme créé sur le moniteur,
la valeur seuil de luminosité est fixée par un utilisateur sur la base de l'histogramme, de l'image binarisée et de l'image ultrasonore affichés sur le moniteur.

18. Appareil de diagnostic par ultrasons selon l'une des revendications 11 à 17, comprenant en outre :
une unité de génération de diagramme schématique du sein (63) qui génère un diagramme schématique du sein sur lequel sont tracés plusieurs emplacements prédéterminés et qui affiche le diagramme schématique sur le moniteur.

19. Appareil de diagnostic par ultrasons selon la revendication 18, comprenant en outre :
une unité de guidage de l'imagerie (64) qui guide l'utilisateur pour qu'il réalise l'imagerie des images ultrasonores à la pluralité d'emplacements prédéterminés.

20. Appareil de diagnostic par ultrasons selon l'une des revendications 11 à 19,
l'unité de calcul du rapport de la région composant le tissu glandulaire (38) stocke le rapport calculé de la région composant le tissu glandulaire dans une étiquette associée à l'image ultrasonore.

21. Appareil de diagnostic par ultrasons selon l'une des revendications 11 à 20,
l'unité de calcul du rapport de la région du tissu glandulaire (38) affiche, sur le moniteur (33), un rapport antérieur de la région du tissu glandulaire calculé sur la base de l'image échographique antérieure du sujet, ainsi qu'un rapport récent de la région du tissu glandulaire calculé sur la base de l'image échographique la plus récente du sujet.

22. Appareil de diagnostic par ultrasons selon l'une des revendications 11 à 21, comprenant en outre :
une sonde à ultrasons (2) ; et
une unité de génération d'images (31) qui génère l'image ultrasonore obtenue par imagerie du sein du sujet en transmettant et en recevant un faisceau ultrasonore vers et depuis le sujet à l'aide de la sonde ultrasonore.

23. Méthode de commande mise en oeuvre par ordinateur pour un appareil d'analyse d'images ultrasonores, la méthode comprenant :
détecter (S3) une région de la glande mammaire à partir d'une image ultrasonore obtenue par imagerie d'un sein d'un sujet ;
extraire (S4) d'une région de composants de tissus glandulaires comprenant des canaux mammaires, des lobules et des stromas périphériques dans la région de la glande mammaire ; et
calculer un rapport entre la région du composant du tissu glandulaire et la région de la glande mammaire.
